(19) Europäisches Patentamt
European Patent Office
Office européen des brevets

(11) **EP 3 820 527 B1**

(12) **EUROPÄISCHE PATENTSCHRIFT**

(45) Veröffentlichungstag und Bekanntmachung des Hinweises auf die Patenterteilung:
**23.08.2023   Patentblatt 2023/34**

(21) Anmeldenummer: **19700185.2**

(22) Anmeldetag: **10.01.2019**

(51) Internationale Patentklassifikation (IPC):
*A61K 47/26* (2006.01)      *A61K 9/107* (2006.01)
*A61K 31/12* (2006.01)      *A61K 36/185* (2006.01)

(52) Gemeinsame Patentklassifikation (CPC):
**A61K 9/0095; A61K 9/1075; A61K 31/05;
A61K 31/12; A61K 36/185; A61K 36/484;
A61K 36/9066; A61K 47/26; A61P 3/00;
A61P 3/04; A61P 3/06; A61P 3/10; A61P 25/16;
A61P 25/28; A61P 29/00;**          (Forts.)

(86) Internationale Anmeldenummer:
**PCT/EP2019/050536**

(87) Internationale Veröffentlichungsnummer:
**WO 2020/011402 (16.01.2020 Gazette 2020/03)**

(54) **XANTHOHUMOL-SOLUBILISAT**

XANTHOHUMOL SOLUBILISATE

SOLUBILISAT DE XANTHOHUMOL

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorität: **11.07.2018   PCT/EP2018/068801
11.07.2018   PCT/EP2018/068731
11.07.2018   PCT/EP2018/068729**

(43) Veröffentlichungstag der Anmeldung:
**19.05.2021   Patentblatt 2021/20**

(73) Patentinhaber: **Aquanova AG
64295 Darmstadt (DE)**

(72) Erfinder: **BEHNAM, Dariush
64380 Roßdorf (DE)**

(74) Vertreter: **Tesch, Sabine
Augspurger Tesch Friderichs
Patent- und Rechtsanwälte PartG mbB
Kaiserstraße 39
55116 Mainz (DE)**

(56) Entgegenhaltungen:
EP-A1- 1 431 385          EP-A1- 2 018 869
WO-A1-2019/011415          DE-A1-102006 062 264

DE-U1-202012 012 130      US-A1- 2016 008 298
US-A1- 2016 081 975

• DATABASE EMBASE [Online] ELSEVIER SCIENCE PUBLISHERS, AMSTERDAM, NL; 1. September 2017 (2017-09-01), KHAYYAL M T: "Novel formulations of Curcumin, Boswellia and Xanthohumol extracts markedly enhance their individual and combined anti-inflammatory activity", XP002783955, Database accession no. EMB-621379886 & ZEITSCHRIFT FUR PHYTOTHERAPIE 20170901 HIPPOKRATES VERLAG GMBH NLD, Bd. 38, Nr. Supplement 1, 1. September 2017 (2017-09-01), ISSN: 1438-9584

• ALEXA KOCHER ET AL: "The oral bioavailability of curcuminoids in healthy humans is markedly enhanced by micellar solubilisation but not further improved by simultaneous ingestion of sesamin, ferulic acid, naringenin and xanthohumol", JOURNAL OF FUNCTIONAL FOODS, Bd. 14, 1. April 2015 (2015-04-01), Seiten 183-191, XP055435032, NL ISSN: 1756-4646, DOI: 10.1016/j.jff.2015.01.045

• **ZAMZOW DANIEL R ET AL: "Xanthohumol improved cognitive flexibility in young mice", BEHAVIOURAL BRAIN RESEARCH, Bd. 275, 1. September 2014 (2014-09-01), Seiten 1-10, XP029077816, ISSN: 0166-4328, DOI: 10.1016/J.BBR.2014.08.045**

(52) Gemeinsame Patentklassifikation (CPC): (Forts.)
**A61P 35/00; A61P 37/00**

**Beschreibung**

Die Erfindung betrifft ein wasserfreies Solubilisat mit Xanthohumol gemäß Anspruch 1. Des Weiteren betrifft die Erfindung ein Fluid enthaltend ein derartiges Solubilisat und eine Kapsel gefüllt mit einem solchen Solubilisat beziehungsweise Fluid.

Xanthohumol ist ein natürlich in Hopfen vorkommendes Flavonoid. Dabei handelt sich um ein prenyliertes Pflanzenpolyphenol, das den Chalkonen zugeordnet wird und bisher ausschließlich im Hopfen nachgewiesen werden konnte. Dabei weisen die Bitterhopfensorten einen deutlich höheren Gehalt an Xanthohumol auf als Aromasorten.

In der europäischen Patentanmeldung EP 1 431 385 A1 werden ein Xanthohumol-haltiges Getränk und ein Verfahren zu dessen Herstellung beschrieben. Beispielsweise kann dafür eine Xanthohumol-Formulierung mit Tween 60 hergestellt werden, indem Tween 60 erwärmt und Xanthohumol bei einer Temperatur von 95°C eingerührt wird.

In Tests zeigte sich Xanthohumol als wirksam gegen die Entstehung und Entwicklung von Krebszellen. In Laborversuchen konnte zudem festgestellt werden, dass Xanthohumol die Nervenzellen des Gehirns schützen kann und dadurch möglicherweise helfen könnte, bei Erkrankungen wie Alzheimer oder Parkinson den Krankheitsverlauf zu verlangsamen.

Beispielsweise wird unter http://www.besserlaengerleben.at/gesund-und-fit/hopfen-hilft-gegen-cholesterin-und-blutzucker.html über Untersuchungen berichtet, nach denen Xanthohumol den Plasmaspiegel bei PCSK9 zu senken scheint, einem Protein, das eine wichtige Rolle bei den Cholesterinwerten spielt. Eine Senkung der PCSK9-Werte könnte den Abbau des LDL-Cholesterins aus dem Blut verbessern. Wissenschaftler der Oregon State University haben nachgewiesen, dass die Einnahme großer Mengen von Xanthohumol bei Versuchstieren zu Verbesserungen beim metabolischen Syndrom und einer verringerten Gewichtszunahme führen kann. Diese Forschungsergebnisse könnten zu neuen Behandlungsansätzen bei Fettleibigkeit, hohen Cholesterinwerten und erhöhtem Blutzucker führen. Die Kombination dieser gesundheitlichen Probleme, bekannt als das metabolische Syndrom, gehören heute mit Herz-Kreislauf-Erkrankungen und Typ-2-Diabetes in den Industrieländern zu den häufigsten Todesursachen.

Xanthohumol kommt natürlich im Hopfen und somit in Bier vor. Die höchsten in der Studie eingesetzten Mengen würden beim Menschen einer Dosis von 350 Milligramm pro Tag für eine Person entsprechen. Dieser Wert übersteigt jedoch deutlich jenen, der durch eine normale Nahrungsaufnahme erreicht werden kann. Eine Einnahme über ein Nahrungsergänzungsmittel wäre jedoch theoretisch problemlos möglich.

Als Nahrungsergänzungsmittel sind derzeit Hopfenextrakte kommerziell erhältlich. Allerdings hat es sich gezeigt, dass die Bioverfügbarkeit des Xanthohumols bei oraler Aufnahme von Hopfenextrakten nur gering ist.

Es ist daher eine Aufgabe der Erfindung, eine Formulierung für Xanthohumol bereitzustellen, die eine verbesserte Bioverfügbarkeit gegenüber nativem Xanthohumol aus Hopfenextrakten zeigt.

Es ist eine weitere Aufgabe der Erfindung, eine Formulierung für Xanthohumol bereitzustellen, die eine verbesserte antiinflammatorische Wirkung im Vergleich zu nativem Xanthohumol erreicht, ohne dass der Wirkstoff Xanthohumol chemisch verändert wird.

Diese Aufgaben wird mit Hilfe der Erfindung in überraschend einfacher Weise gelöst mit einem Solubilisat nach Anspruch 1.

Die Erfindung stellt ein wasserfreies Solubilisat bestehend aus Xanthohumol mit einem Anteil von kleiner oder gleich 35 Gew.-%, bevorzugt kleiner oder gleich 15 Gew.-%, besonders bevorzugt 2 Gew.-% bis 12 Gew.-% und zumindest einem Emulgator mit einem HLB-Wert im Bereich zwischen 13 und 18, welcher insbesondere ausgewählt ist aus der Gruppe, welche Polysorbat 80, Polysorbat 20, Zuckerester aus Speisefettsäuren (E 473) und Phospholipide, insbesondere Lecithin, und Mischungen von zumindest zwei der genannten Emulgatoren umfasst, und bis zu 35 Gew.-%, bevorzugt bis zu 20 Gew.-%, besonders bevorzugt bis zu 15 Gew.-% Ethanol und optional bis zu 25 Gew.-%, bevorzugt bis zu 10 Gew.-% Glycerin. Wie unten näher ausgeführt wird, weist das erfindungsgemäße Solubilisat eine verbesserte Bioverfügbarkeit gegenüber nativem Xanthohumol aus Hopfenextrakten auf sowie eine verbesserte antiinflammatorische Wirkung im Vergleich zu nativem Xanthohumol.

Bei dem erfindungsgemäßen Solubilisat handelt es sich um ein kolloidal formuliertes Gemisch als Kapselfüllung, in welchem Xanthohumol als bioaktiver Wirkstoff enthalten ist. Mit der Erfindung kann bereits alleine aus Emulgator und Xanthohumol ein Solubilisat mit auch auch unter physiologischen Bedingungen stabilen Micellen geschaffen werden. Das Solubilisat kann jedoch auch ein Gemisch sein, welches neben Xanthohumol hauptsächlich aus technischen Hilfsstoffen, Trägerstoffen, Füllstoffen und Stabilisatoren besteht. Das erfindungsgemäße Soluilisat ist wasserfrei beziehungsweise nicht wässrig. Es ist ausgelegt darauf, als Kapselfüllung verwendet zu werden. Insbesondere ist es zwar flüssig, aber kein Getränk.

Als Voraussetzung für die Absorption von fettlöslichen Mikronährstoffen durch den menschlichen oder tierischen Körper gilt die Bildung von sogenannten "physiologischen Mischmicellen" mit Hilfe von Gallensäuren, Gallensalzen und Enzymen aus dem Verdauungstrakt. Das erfindungsgemäße Solubilisat weist daher analog zu diesem Naturprinzip und vor dem Hintergrund der Optimierung der Bioverfügbarkeit des Xanthohumols eine stabile micellare Struktur auf. Die Verbesserung der Bioverfügbarkeit kann auf diesem Wege gerade dann erreicht werden, wenn sich die Micelle unter physiologischen Bedingungen - das heißt bei einer Temperatur von 37° C und pH 1,1 - als stabil erweist und unbeschadet den Dünndarm erreicht.

Aufgrund des hohen Anteils an Xanthohumol sieht die Erfindung in einer vorteilhaften Ausgestaltung vor, dass das Solubilisat als Quelle für Xanthohumol einen ethanolischen Auszug der Hartharze aus Hopfen enthält, wobei Xanthohumol in diesem Auszug in einer Konzentration im Bereich zwischen 60 Gew.-% und 95 Gew.-%, bevorzugt auf eine Konzentration im Bereich von 65 Gew.-% bis 85 Gew.-% vorliegt. Insbesondere die unten näher erläuterten Produkte "Xantho-Flav pur" und "Xantho-Flav" können im Rahmen der Erfindung als Xanthohumolquelle verwendet werden.

In einer bevorzugten Ausführungsform enthält das Solubilisat gemäß der Erfindung als Emulgator Polysorbat 80 oder Polysorbat 20 oder eine Mischung aus Polysorbat 20 und Polysorbat 80. Die Erfindung schafft dabei vorteilhafterweise die Möglichkeit, für die Herstellung stabiler Micellen von Xanthohumol auch unter physiologischen Bedingungen (pH 1,1 und 37°C) den Emulgator beziehungsweise die Emulgatorzusammensetzung wählen zu können. So kann als Emulgator auch zumindest ein Zuckerester einer Speisefettsäure oder eine Mischung mehrerer Zuckerester von Speisefettsäuren verwendet werden. Des Weiteren hat der Erfinder herausgefunden, dass auch die Mischung von Polysorbat 80 oder Polysorbat 20 oder einer Mischung aus Polysorbat 80 und Polysorbat 20 mit zumindest einem Zuckerester einer Speisefettsäure als Emulgator im Rahmen der Erfindung eingesetzt werden kann. Zudem kann eine Mischung zumindest eines Phospholipids, beispielsweise Lecithin, mit zumindest einem Zuckerester einer Speisefettsäure als Emulgator im Rahmen der Erfindung verwendet werden.

So bietet die Erfindung den Vorteil, die Zusammensetzung abstimmen zu können je nachdem, welche weiteren Komponenten im Solubilisat enthalten sein sollen und in welchen Mengenverhältnissen, dessen Eigenschaften im Hinblick auf die Bioverfügbarkeit, Lagerstabilität und Wechselwirkungen mit dem Material von Kapseln, in welchen das Solubilisat zur oralen Verwendung bereitgestellt werden soll,

Es hat sich herausgestellt, dass je nachdem, wie viel Xanthohumol solubilisiert werden soll, und insbesondere auch in Abhängigkeit von der Frage, ob weitere Wirkstoffe zusätzlich zu Xanthohumol mizelliert werden sollen, das Massenverhältnis von Emulgator, insbesondere von Polysorbat 80, zu Xanthohumol im Bereich zwischen 30:1 und 3:1, bevorzugt im Bereich zwischen 25:1 und 5:1, bevorzugt im Bereich zwischen 9,8:1 bis 6,6:1 angepasst werden kann.

Der Emulgatoranteil, insbesondere der Polysorbatanteil, liegt gemäß der Erfindung dazu bei mindestens 45 Gew.-%, bevorzugt im Bereich zwischen 60 Gew-% und 95 Gew-%, besonders bevorzugt im Bereich zwischen 73 Gew-% und 90 Gew-%. Je nachdem, wie viel Emulgator für einen bestimmten Verwendungszweck des Solubilisats eingesetzt werden kann, bietet die Erfindung die Möglichkeit, dass das Solubilisat bis zu 35 Gew.-%, bevorzugt bis zu 20 Gew.-%, besonders bevorzugt bis zu 15 Gew.-% Ethanol enthält. Durch Zugabe von Ethanol kann der Anteil von Polysorbat reduziert werden, was im Hinblick auf den ADI-Wert für Polysorbat einen Vorteil darstellt.

Für die Ausbildung stabiler Micellen kann es je nachdem, welche Wirkstoffe in welcher Menge solubilisiert werden sollen hilfreich sein, dass das Solubilisat bis zu 25 Gew.-%, bevorzugt bis zu 10 Gew.-% Glycerin enthält. Auch durch Zugabe von Glycerin kann der Anteil von Polysorbat reduziert werden.

Die Solubilisate gemäß der Erfindung haben auch unter den physiologischen Bedingungen einer Magenpassage eine enge Partikelgrößenverteilung mit kleinen mittleren Partikelgrößen, bevorzugt reicht die Durchmesserverteilung der Micellen in einer Verdünnung des Solubilisats mit destilliertem Wasser im Verhältnis 1:500 bei pH 1,1 und 37°C von etwa $d_{10}$=7O nm bis etwa $d_{90}$=16O nm. Diese Werte wurden anhand einer Intensitätsverteilung ermittelt. Einzelheiten zur Partikelgrößenanalyse der Micellen der Solubilisate werden unten erläutert.

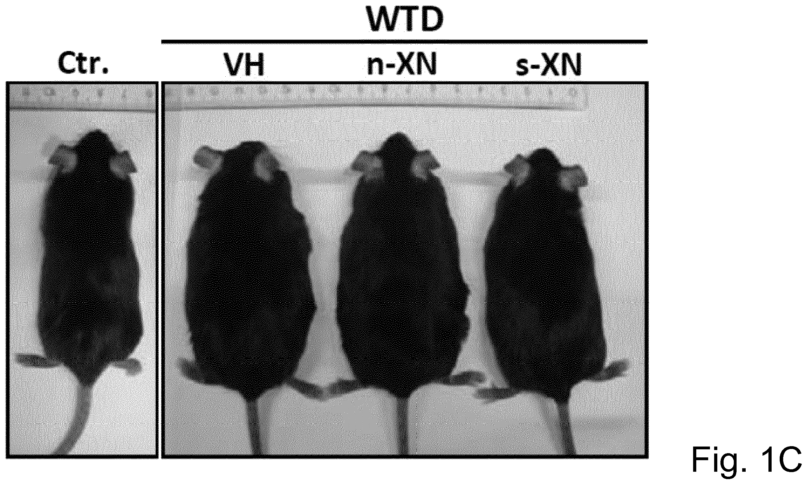

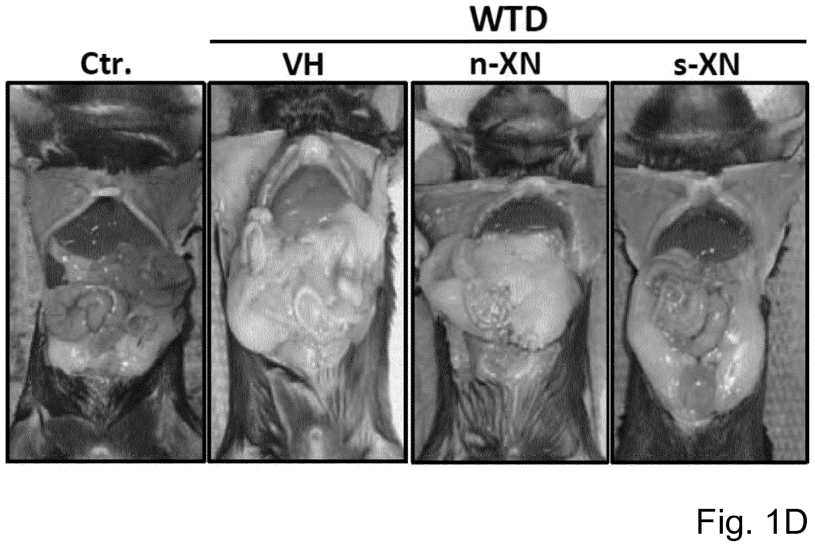

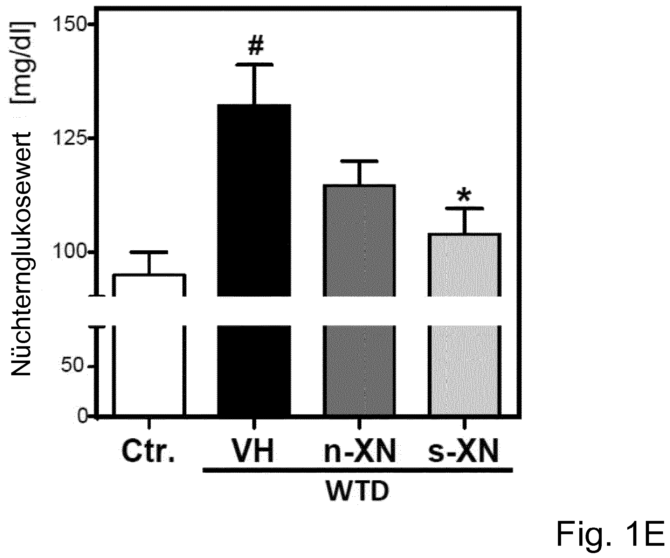

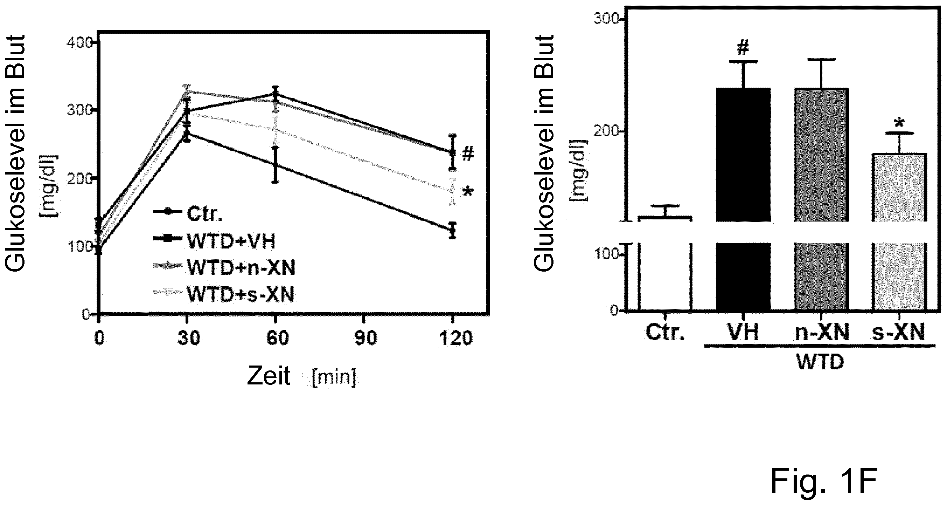

Die Erfindung stellt vorteilhafterweise Solubilisate mit sehr guter entzündungshemmenden Eigenschaften zur Verfügung. Die antiinflamatorische Aktivität des Xanthohumol-Solubilisats gemessen als Konzentration von C- reaktivem Protein (CRP) im Blutserum arthritischer Ratten nach einmaliger Gabe des Solubilisats in einer Dosierung von 5 mg/kg Körpergewicht liegt im Bereich von etwa 2500 pg/mL bis etwa 2700 pg/mL und damit deutlich niedriger als die Konzentration von C-reaktivem Protein (CRP) im Blutserum arthritischer Ratten nach einmaliger Gabe von nativem Xanthohumol in einer Dosierung von 5 mg/kg Körpergewicht, welche im Bereich von etwa 3300 pg/mL bis etwa 3700 pg/mL liegen. Dazu wird auf die beigefügte Figur 1c verwiesen und die Beschreibung dazu im Folgenden.

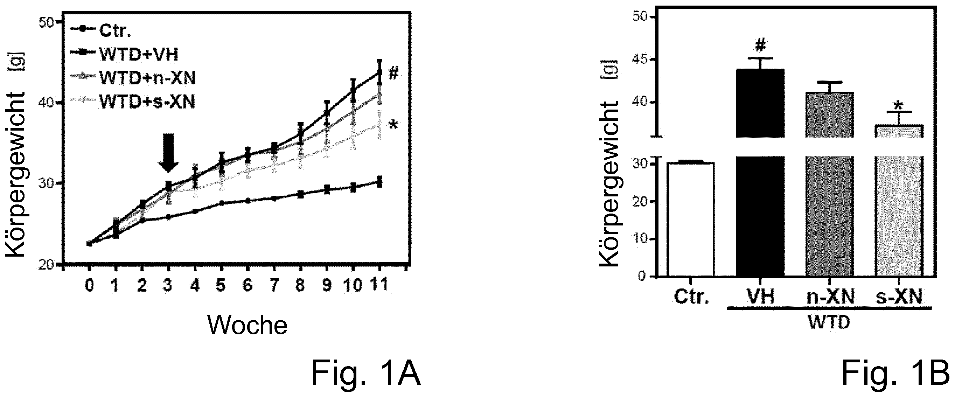

Zum Vergleich liegt die antiinflamatorische Aktivität eines Solubilisats mit Xanthohumol und Curcumin gemessen als Konzentration von C-reaktivem Protein (CRP) im Blutserum arthritischer Ratten nach einmaliger Gabe des Solubilisats in einer Dosierung von Xanthohumol und Curcumin von jeweils 5 mg/kg Körpergewicht im Bereich von etwa 2000 pg/mL bis etwa 2400 pg/mL und damit deutlich niedriger als die Konzentration von C-reaktivem Protein (CRP) im Blutserum arthritischer Ratten nach einmaliger Gabe von nativem Xanthohumol und nativem Curcumin in einer Dosierung von jeweils 5 mg/kg Körpergewicht, welche im Bereich von etwa 3200 pg/mL bis etwa 3600 pg/mL liegen. In diesem Zusammenhang wird auf die beigefügte Figur 1b verwiesen und die zugehörige Beschreibung unten.

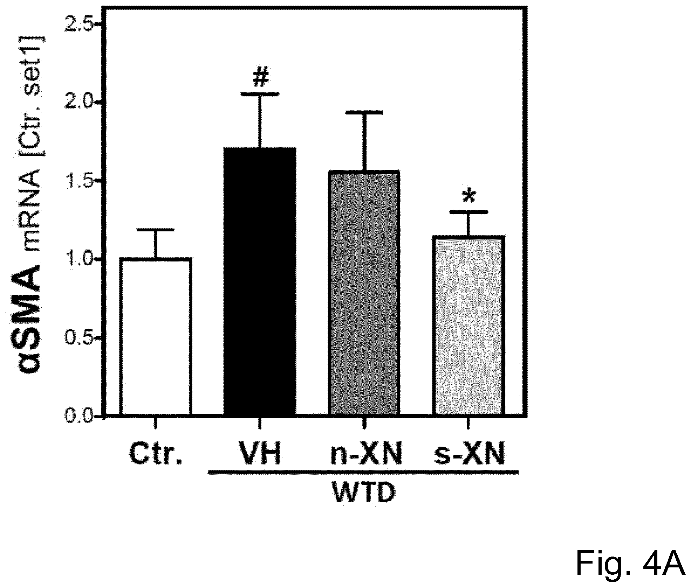

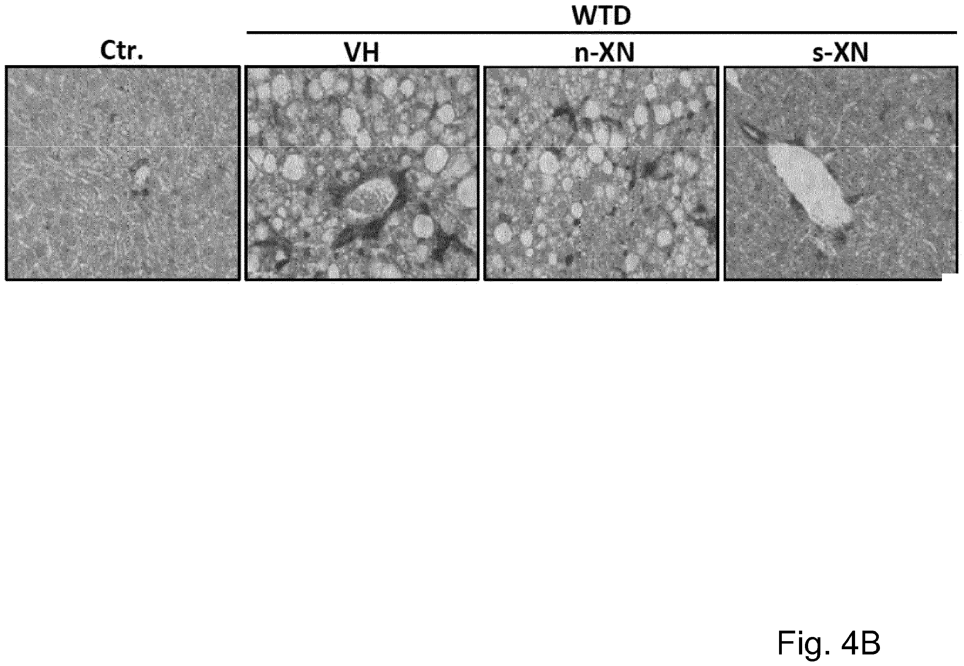

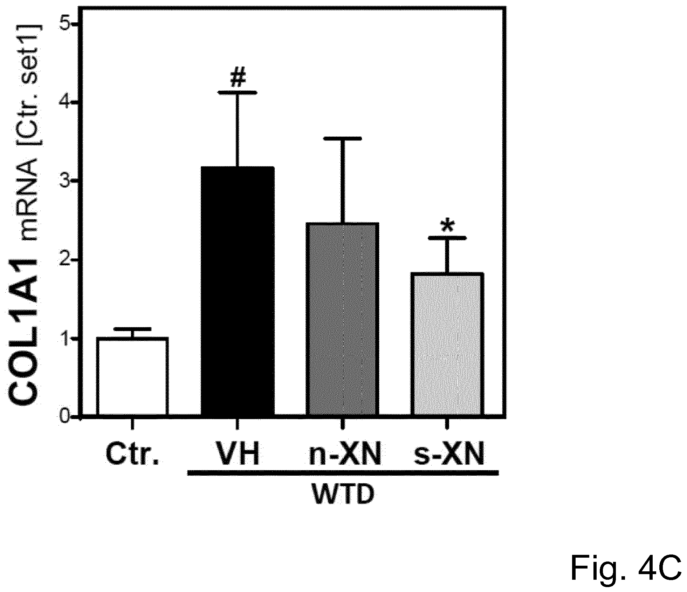

Die antiinflamatorische Wirkung eines erfindungsgemäßen Xanthohumol-Solubilisats gemessen als Konzentration von Myeloperoxidase (MPO) im Blutserum arthritischer Ratten liegt nach einmaliger Gabe des Solubilisats in einer Dosierung von 5 mg/kg Körpergewicht im Bereich von etwa 800 mU/mL bis etwa 900 mU/mL und damit deutlich niedriger als die Konzentration von Myeloperoxidase (MPO) im Blutserum arthritischer Ratten nach einmaliger Gabe von nativem Xanthohumol in einer Dosierung von 5 mg/kg Körpergewicht, welche im Bereich von etwa 1100 mU/mL bis etwa 1200 mU/mL liegen. In diesem Zusammenhang wird auf die beigefügte Figur 4 verwiesen und die zugehörige Beschreibung unten.

Die Enzymeinheit (U) ist eine heute mittlerweile durch das Katal abgelöste Einheit zur Angabe der Enzymaktivität. Da sich bei Verwendung des Katal die Zahlenwerte ändern, wird die Enzymeinheit (U) in Medizin und klinischer Chemie weiterhin genutzt. Eine Enzymeinheit U entspricht einem Mikro-Mol Substratumsatz pro Minute.

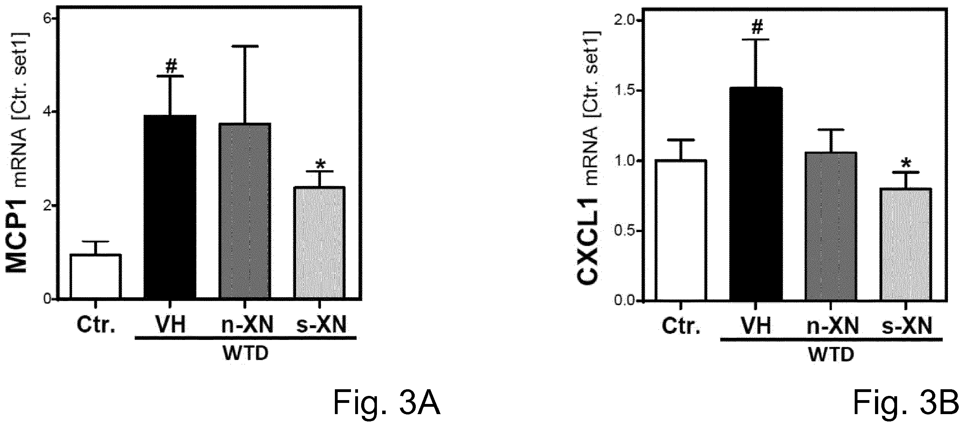

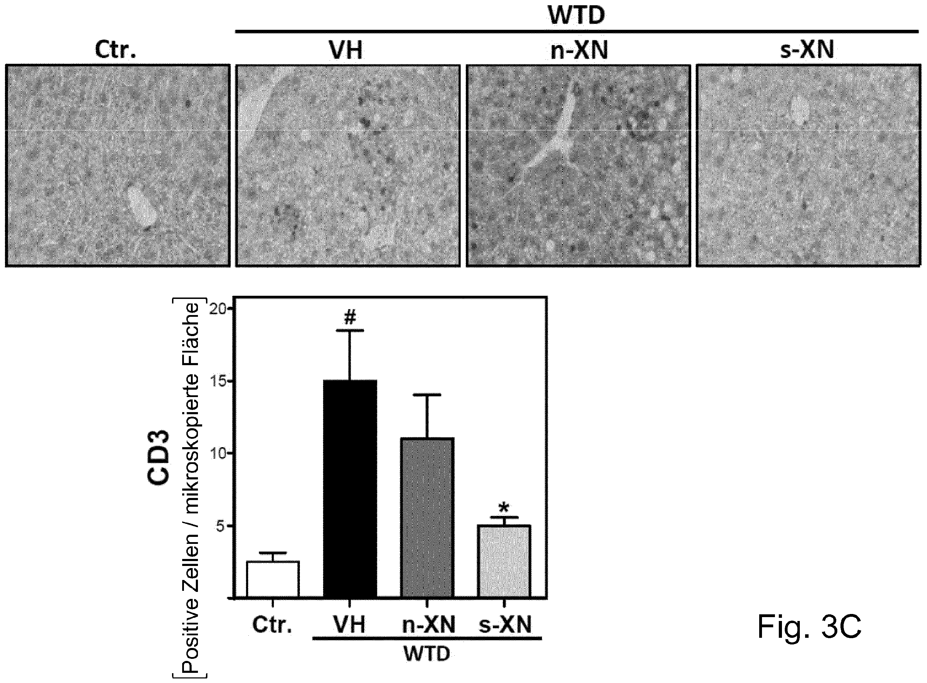

Die antiinflamatorische Wirkung eines Solubilisats mit Xanthohumol und Curcumin gemessen als Konzentration von Myeloperoxidase (MPO) im Blutserum arthritischer Ratten nach einmaliger Gabe des Solubilisats in einer Dosierung von Xanthohumol und Curcumin von jeweils 5 mg/kg Körpergewicht liegt im Bereich von etwa 550 mü/mL bis etwa 600 mU/mL und damit deutlich niedriger als die Konzentration von Myeloperoxidase (MPO) im Blutserum arthritischer Ratten nach einmaliger Gabe von nativem Xanthohumol und nativem Curcumin in einer Dosierung von jeweils 5 mg/kg Körpergewicht, welche im Bereich von etwa 1100 mU/mL bis etwa 1300 mU/mL liegen. In diesem Zusammenhang wird auf die beigefügte Figur 3 verwiesen und die zugehörige Beschreibung unten.

Einen Hinweis auf die im Vergleich zu nativem Xanthohumol beziehungsweise nicht gemäß der Erfindung mizellierten Zusammensetzungen von Xanthohumol mit Curcumin und/oder Glavonoid verbesserte Bioverfügbarkeit gibt die messtechnisch deutlich einfacher zugängliche Bestimmung der Trübung des Solubilisats. Die Trübung des Solubilisats ist vorzugsweise infolge der erfindungsgemäßen Formulierung kleiner als 100 FNU, bevorzugt kleiner als 50 FNU, ist gemessen durch Streulichtmessung mit Infrarotlicht nach den Vorschriften der Norm ISO 7027 bei einer Verdünnung des Solubilisats im Verhältnis 1:50 in Wasser bei pH 1,1 und 37°C.

Die Korrelation zwischen der Beladung einer Micelle mit Xanthohumol und ihrer Stabilität verläuft dabei umgekehrt proportional. Die Erhöhung der Beladung führt entsprechend zu größerer Instabilität der Micelle. Ausschließlich die intakten Micellen führen zu einer Erhöhung der Bioverfügbarkeit. Wie die engen Partikelgrößenverteilungen und auch die äußerst geringe Trübung der erfindungsgemäßen Solubilisate zeigen, schafft die Erfindung den großen Vorteil, hochbeladene stabile Micellen bereit zu stellen, die auch unter physiologischen Bedingungen stabil sind, und daher eine hohe Bioverfügbarkeit des Xanthohumols erreichen.

Um die orale Anwendung des erfindungsgemäßen Solubilisats in für den Konsumenten beziehungsweise Patienten einfacher und angenehmer Weise zu ermöglichen, stellt die Erfindung zudem eine Kapsel gefüllt mit einem oben beschriebenen wasserfreien Solubilisat zur Verfügung, wobei die Kapsel als Weichgelatinekapsel oder Hartgelatinekapsel oder als weiche, gelatinefreie Kapsel oder als harte, gelatinefreie Kapsel, insbesondere als Cellulosekapsel, ausgebildet ist.

Das erfindungsgemäße Solubilisat kann im Rahmen der Erfindung zudem in andere Fluide, insbesondere Flüssigkeiten eingearbeitet werden. Dabei bleiben die Wirkstoffgefüllten kleinen Micellen erhalten. Somit stellt die Erfindung auch ein Fluid enthaltend ein oben beschriebenes Solubilisat zur Verfügung, wobei das Fluid aus der Gruppe ausgewählt ist, welche Lebensmittel, Getränke, Kosmetika und pharmazeutische Produkte umfasst. Insbesondere kann das Fluid eine wässrige Verdünnung des Solubilisats umfassen.

Die Erfindung stellt des Weiteren ein Verfahren zum Herstellen eines oben beschriebenen Solubilisats mit Xanthohumol mit folgenden Schritten bereit:

a) Vorlegen zumindest eines Emulgators mit einem HLB- Wert im Bereich zwischen 13 und 18, welcher insbesondere ausgewählt ist aus der Gruppe, welche Polysorbat 80, Polysorbat 20, Zuckerester aus Speisefettsäuren (E 473) und Phospholipide, insbesondere Lecithin, und Mischungen von zumindest zwei der genannten Emulgatoren umfasst,
b) Zugabe von Ethanol,
c) Erwärmen auf eine Temperatur bis zu 85°C, bevorzugt bis zu 80°C, unter Mischen beispielsweise durch Rühren,
d) Zugabe eines ethanolischen Auszugs der Hartharze aus Hopfen, insbesondere Xantho-Flav pur-Pulver und/oder Xantho-Flav-Pulver, unter Mischen beispielsweise durch Rühren,

wobei in Schritt d) eine Erwärmung auf eine Temperatur im Bereich von 81°C bis 90°C, besonders bevorzugt auf eine Temperatur im Bereich von 83°C und 87°C erfolgt.

Soll das Solubilisat gemäß der ersten Verfahrensvariante auch Ethanol enthalten, sieht die Erfindung vor, vor Schritt b) einen

bl) Lösen des ethanolischen Auszugs der Hartharze aus Hopfen, insbesondere Xantho-Flav pur-Pulver, in Ethanol unter Erwärmung auf eine Temperatur im Bereich von 40°C bis 62°C, bevorzugt auf eine Temperatur im Bereich von 45°C bis 57°C,

besonders bevorzugt auf eine Temperatur im Bereich von 48°C und 52°C,

durchzuführen.

In jedem Fall kann in Schritt c) bei Bedarf zur Unterstützung des Mischungs- beziehungsweise Lösungsvorgangs eine Erwärmung auf eine geringere Temperatur im Bereich von 40°C bis 62°C, bevorzugt auf eine Temperatur im Bereich von 45°C bis 57°C, besonders bevorzugt auf eine Temperatur im Bereich von 48°C und 52°C, erfolgen.

Des Weiteren kann im Rahmen der Erfindung je nachdem, welche Emulgatorzusammensetzung für den betreffenden Anwendungsfall ausgewählt wurde, in Schritt b) eine Zugabe eines Phospholipids, insbesondere eine Zugabe von Lecithin, zusammen mit Ethanol erfolgen. In Schritt b) ist es auch möglich, zusammen mit Ethanol Glycerin und/oder Wasser zuzugeben.

Die Erfindung wird nachfolgend anhand von Ausführungsbeispielen näher erläutert. Dabei wurden die folgenden Komponenten verwendet.

### Xanthohumol

Als Xanthohumol-Quelle wurden die Produkte "Xantho-Flav pur" oder "Xantho-Flav 75 % (65-85 %)" der Marke "Hopsteiner" der Simon H. Steiner, Hopfen, GmbH, Mainburg, Deutschland verwendet. Bei beiden handelt es sich um ein Naturprodukt, das aus Hopfen hergestellt wird. Der aktive Inhaltsstoff ist das Hopfen-Polyphenol Xanthohumol. Es handelt sich um ein gelbfarbenes Pulver mit einem Xanthohumol-Gehalt nach Herstellerangaben von mindestens 85 % bei "Xantho-Flav pur". Der Xanthohumol-Gehalt von "Xantho-Flav 75 % (65 - 85 %)" betrug bei den im Folgenden vorgestellten Ausführungsbeispielen mindestens 70 %. Die Konzentrationen an Xanthohumol und Isoxanthohumol in "Xantho-Flav pur" werden vom Hersteller nach der UVspektrophotometrischen Analyse oder nach HPLC EBC 7.8 über externen Kalibrierstandard reines XN (370 nm) bzw. IX (290 nm) quantifiziert. "Xantho-Flav pur" enthält das prenylierte Flavonoid Xanthohumol in sehr hoher Konzentration. Für die Ausführungsbeispiele im Rahmen der vorliegenden Anmeldung wurde "Xantho-Flav pur" der Batchnummer 9432 verwendet.

### Polysorbat 80

Als Quelle für Polysorbat 80 wurde das Material "TEGO SMO 80 V FOOD" mit dem Spezifikationscode "K04 EU-FOOD" der Evonik Nutrition & Care GmbH, Essen, Deutschland, verwendet. Das Produkt entspricht den EU-Anforderungen des Lebensmittelzusatzstoffes E 433. In den im Folgenden beschriebenen Ausführungsbeispielen können alternativ zu dem erwähnten TEGO SMO 80 V von Evonik als Polysorbat 80 auch TEGO SMO 80 V von der InCoPA Gmbh, Illertissen, Deutschland oder Crillet 4/Tween 80-LQ-(SG) von der CRODA GmbH, Nettetal, Deutschland oder Lamesorb SMO 20 sowie Kotilen-O/1 VL von Univar oder der Kolb Distributions AG, Hedingen, Schweiz verwendet werden.

### Zuckerester von Speisefettsäuren (E473)

Als Zuckerester von Speisefettsäuren (E 473) können einzelne Zuckerester von Speisefettsäuren (E 473) oder Mischungen von zumindest zwei Zuckerestern von Speisefettsäuren verwendet werden. Geeignete Zuckerester von Speisefettsäuren sind insbesondere Sucrose-mono-Laurat, Sucrose-di-Laurat, Sucrose-mono-Palmitat, Sucrose-di-Palmitat, Sucrose-mono-Stearat und Sucrose-di-Stearat. Für die im Folgenden beschriebenen Ausführungsbeispiele wurden die Produkte "Ryoto L-1695", "Ryoto P-1670" und "Ryoto M- 1695" des Herstellers Mitsubishi-Kagaku Foods Corporation verwendet. Des Weiteren wurde als Zuckerester aus Speisefettsäuren (Sucroseester, E 473) das Produkt "DUB SE 16 P" des Herstellers Stearinerie Dubois verwendet. Auch das Produkt "DUB SE 16 S" kann zum Herstellen erfindungsgemäßer Solubilisate von Xanthohumol eingesetzt werden.

Als **Phospholipid** wurde Lecithin in Form des Produktes "Epikuron® 135 F" oder "Epikuron® 135 F IP (GMOfrei)(35 % bzw. 32 % Phosphatidylcholin in Sojaöl)" der Cargill Inc. Verwendet.

### Glycerin

Als Glycerin wurde im Rahmen der vorliegenden Anmeldung das Produkt "Glycamed 99,7 %" der Glaconchemie GmbH, Merseburg, Deutschland, verwendet. Nach Herstellerangaben liegt der Glycerin-Gehalt dieses Produkts bei mindestens 99,5 %.

### Ethanol

Ethanol wurde im Rahmen der vorliegenden Anmeldung von der Berkel pfälzische Spritfabrik GmbH & Co. KG bezogen. Gemäß der Spezifikation für "Neutralalkohol unvergällt" 1411U versteuert" beträgt der Gehalt an Ethanol dieses Produkts etwa 94 +/- 1% %.

Als **Wasser** wurde destilliertes Wasser verwendet.

Die Partikelgrößenanalysen der Micellen in wässrigen Verdünnungen erfindungsgemäßer Solubilisate wurden gemessen nach dem Prinzip der dynamischen Lichtstreuung mit Laserlicht der Wellenlänge 780 nm. Die Partikelgrößenmessungen wurden mit dem ParticleMetrix NANOFLEX Rückstreu-Teilchenanalysator durchgeführt. Das Messprinzip beruht auf der dynamischen Lichtstreuung (DLS) in einer 180° Heterodyn-Rückstreuanordnung. Bei dieser Geometrie wird zum gestreuten Licht ein Teil des Laserstrahls dazu gemischt (Heterodyn-Technik). Wegen des geringen Lichtweges von 200 Mikrometer bis 300 Mikrometer in der Probe ist die Rückstreuung für absorbierende und hochkonzentrierte Proben von Vorteil. Die Heterodyn-Technik wirkt sich verstärkend auf das Signal/Rausch-Verhältnis und auf die Empfindlichkeit des Sub-lOOnm-Bereiches aus.

Das Laserlicht wird in die Y-Gabel einer Lichtfaser eingekoppelt. Zurück kommen in derselben Faser das am Saphirfenster der Probenkammer teilreflektierte Laserlicht und das von der Probe rückwärts gestreute Licht. Der Detektor im zweiten Ast der Y-Gabel nimmt die miteinander interferierenden Signale auf. Eine schnelle Fouriertransformations-Auswertung zerlegt die fluktuierenden Streulichtanteile in ein frequenzabhängiges sogenanntes "Power-Spektrum". Jeder Frequenzanteil stellt eine Brown'sche Diffusionskonstate dar und ist damit einer Partikelgröße zuzuordnen. Zur Umrechnung in eine Partikelgrößenverteilung wird die Stokes-Einstein-Formel verwendet:

$$D = k \frac{T}{3\pi\eta d_P}$$

In diese Gleichung gehen ein die Diffusionskonstante D, die Boltzmannkonstante k, die Temperatur T, die dynamische Viskosität $\eta$ des Mediums und der Durchmesser $d_P$ der Partikel. Ein Temperatursensor ist im Messgerät probennah in der Nähe des Saphirfensters angebracht.

Zur experimentellen Bestimmung der Trübung der erfindungsgemäßen Solubilisate werden die Trübungsmessgeräte mit einer Standardsuspension kalibriert. Die Anzeige erfolgt somit nicht in Form der gemessenen Lichtintensität, sondern als Konzentration der Kalibriersuspension. Bei der Messung einer beliebigen Suspension bedeutet also die Anzeige, dass die betreffende Flüssigkeit die gleiche Lichtstreuung verursacht wie die Standardsuspension der angezeigten Konzentration. Der international festgelegte Trübungsstandard ist Formazin. Zu den geläufigsten Einheiten gehört die Angabe "FNU", das heißt "Formazine Nephelometrie Units". Dies ist die beispielsweise in der Wasseraufbereitung verwendete Einheit für die Messung bei 90° gemäss den Vorschriften der Norm ISO 7072.

**Vergleichsbeispiel 1**

10 %iges Xantho Flav pur-Solubilisat

Es werden

100 g Xantho Flav pur und
900 g Polysorbat 80
verwendet.

Dazu wird das Xantho Flav pur-Pulver durch Rühren in Polysorbat 80 eingearbeitet. Das Pulver wird dabei mit einer solchen Geschwindigkeit zugegeben, dass es gleichmäßig in den Emulgator eingezogen wird. Unter Erwärmen auf 83 bis 87°C wird weiter homogenisiert. Nachdem ein homogenes Solubilisat erreicht ist, wird auf eine Temperatur unterhalb von 60° abgekühlt. Das Xantho Flav- Solubilisat wird dann abgefüllt und dunkel und kühl, d.h. bei unter 25°C gelagert.

**Ausführungsbeispiel 2**

10 %iges Xantho Flav pur-Solubilisat mit Ethanol

Für diese Variante eines erfindungsgemäßen Xanthohumol-Solubilisats werden

100 g Xantho Flav pur,
150 g Ethanol (96 %ig) Neutralalkohol Sorte 1411U und
750 g Polysorbat 80
verwendet.

Zunächst wird das Xantho Flav pur-Pulver in Ethanol gelöst und dabei auf eine Temperatur im Bereich zwischen 48 und 52°C erwärmt. Es entsteht eine homogene Lösung. Polysorbat 80 wird dann unter Erwärmen auf 83 bis 87°C zur Lösung von Xantho Flav pur in Ethanol zugegeben. Die Zugabe erfolgt mit einer solchen Geschwindigkeit, dass sich die beiden Fluide durch Rühren gut homogenisieren. Das entstehende Solubilisat wird auf unter 60°C abgekühlt und abgefüllt sowie dunkel und kühl, d.h. bei Temperaturen unterhalb von 25°C gelagert.

**Ausführungsbeispiel 2A**

Ein 10 %iges Xantho Flav pur-Solubilisat mit Ethanol kann auch hergestellt werden mit weniger Ethanol und mehr Polysorbat 80.

Für diese Variante eines erfindungsgemäßen Xanthohumol-Solubilisats werden

100 g Xantho Flav pur,
100 g Ethanol (96 %ig) Neutralalkohol Sorte 1411U und
800 g Polysorbat 80
verwendet.

Zunächst wird das Xantho Flav pur-Pulver in Ethanol gelöst und dabei auf eine Temperatur im Bereich zwischen 56 und 60°C erwärmt. Es entsteht eine homogene Lösung. Die weitere Herstellung entspricht Ausführungsbeispiel 2.

**Ausführungsbeispiel 3**

12 %iges Xantho Flav pur-Solubilisat mit Ethanol

Zur Herstellung werden

120 g Xantho Flav pur,
150 g Ethanol (96 %ig) Neutralalkohol Sorte 1411U und
730 g Polysorbat 80
verwendet.

Die Herstellung des 12 %igen Xantho Flav pur-Solubilisats erfolgt genauso wie gemäß Ausführungsbeispiel 2.

Die hergestellten Solubilisate sind dunkelbraun und trotz leichter Viskosität gut fließfähig. In einem Verhältnis von 1:50 gelöst in Wasser bei etwa 40 bis 50°C entsteht eine dunkelgelb bis ockerfarbene, leicht trübe Lösung. Mittels HPLC-Messungen wurde beim 10 %igen Xantho Flav-Solubilisat mit Ethanol, der Gehalt des Xantho Flavs von 10 % in die Micellen eingeschlossen, bestätigt. Über eine Aräometer- Messung bei 20°C wurde die Dichte dieses Solubilisats auf 1 bis 1,1 $g/cm^3$ bestimmt. Der pH-Wert des Solubilisats lag zwischen 5 und 7 bei der im Verhältnis 1:50 mit Wasser zubereiteten Lösung. Die Gesamtkeimzahl war kleiner oder maximal gleich 1.000/g. Hefen und Schimmelpilze lagen bei maximal 100/g und in 1 g Solubilisat konnte kein E.-coli bzw. coliforme Keime nachgewiesen werden gemäß der Methode Ph.Eur. der im Oktober 2014 gültigen Version.

Für das Xanthohumol-Solubilisat mit Ethanol gemäß Ausführungsbeispiel 2 wurden bei einer Verdünnung im Verhältnis 1:50 in Wasser bei einem pH-Wert von 1,1 und einer Temperatur von 37°C, d.h. unter physiologischen Bedingungen, Trübungsmessungen vorgenommen. Der über drei Proben gemittelte Wert beträgt 40,9 FNU, welcher sich aus Einzelwerten von 26,0 FNU, 6,4 FNU und 90,2 FNU berechnet.

Für die Partikelgrößenmessung des Xanthohumol-Solubilisats mit Ethanol gemäß Ausführungsbeispiel 2 wurde dieses zunächst im Verhältnis 1:500 mit destilliertem Wasser verdünnt und unter ständigem Rühren mit einem Magnetrührer und mit Hilfe einer Heizplatte auf 37°C gebracht.

Anschließend wurde der pH-Wert mit 32 %iger Salzsäure auf 1,1 eingestellt. Die Proben wurden im Anschluss sofort vermessen. Die Ergebnisse der Intensitätsvereilung sind in der folgenden Tabelle zusammengestellt.

| $d_{10}$ (nm) | $d_{50}$ (nm) | $d_{90}$ (nm) | $d_{99}$ (nm) |
|---|---|---|---|
| 76,90 | 107,0 | 155,3 | 212,9 |

**Ausführungsbeispiel 4**

Ein erfindungsgemäßes Xanthohumol-Solubilisat kann auch unter Zusatz von Glycerin hergestellt werden.

3,8 %iges Xantho Flav pur-Solubilisat mit Glycerin

Es werden zur Herstellung von 875 g Solubilisat

33,3 g Xantho-Flav pur-Pulver
33,3 g Glycerin 99,5 %
808,4 g Polysorbat 80
verwendet.

Polysorbat 80 und Glycerin werden unter Rühren gemischt und dabei auf eine Temperatur im Bereich von 48 bis 52°C erwärmt, um die Mischung gut zu homogenisieren. Ethanol wird unter derart starkem Rühren in das Polysorbat-Glycerin-Gemisch eingearbeitet, dass sich eine homogene Lösung bildet. Die Temperatur wird dabei konstant gehalten. Dann wird Xanthohumol in die Lösung aus Polysorbat, Glycerin und Ethanol eingearbeitet. Dabei wird die Temperatur auf einen Wert im Bereich zwischen 63 und 67°C erhöht. Es wird derart stark gerührt, dass sich Xanthohumol homogen mit der vorgelegten Lösung verbindet.

Eine weitere Möglichkeit, ein erfindungsgemäßes Solubilisat herzustellen, liegt in der Verwendung von Zuckerestern von Speisefettsäuren als Emulgator.

**Ausführungsbeispiel 5**

10 %iges Xantho Flav-Solubilisat

Es werden

| | |
|---|---|
| 100 g | Xantho-Flav Pulver, |
| 598,5 g | Zuckerester aus Speisefettsäuren (E 473) "Ryoto L-1695" und, |
| 301,5 g | Ethanol 96 %ig Neutralalkohol Sorte 1411U |

verwendet.

Der Zuckerester wird mit Ethanol unter Rühren bei Raumtemperatur im Temperaturbereich von 20°C bis einschließlich 25°C vermischt, so dass sich ein homogenes Gemisch bildet. Dieses wird unter Rühren auf eine Temperatur von maximal 80°C unter Rühren erwärmt. Es wird so lange weiter gerührt, bis eine homogene, transparente Lösung erreicht ist. Dann wird das Xantho-Flav Pulver zugegeben und unter Rühren auf eine Temperatur im Bereich von 83°C bis 97°C weiter erhitzt. Es wird derart stark gerührt, dass sich Xanthohumol homogen mit der vorgelegten Lösung verbindet. Das Solubilisat ist nach Abkühlen auf Raumtemperatur dunkelbraun und transparent. In diesem Zustand wird es abgefüllt und dann gelagert.

**Ausführungsbeispiel 6**

5 %iges Xantho Flav-Solubilisat

Es werden

| | |
|---|---|
| 50 g | Xantho-Flav Pulver, |
| 627 g | Zuckerester aus Speisefettsäuren (E 473) und |
| 323 g | Ethanol 96 %ig Neutralalkohol Sorte 1411U |

verwendet.

Der verwendete Zuckerester aus Speisefettsäuren ist ein Gemisch aus

41 Gew.-% Sucrose-mono-Laurat,
32 Gew.-% Sucrose-mono-Palmitat,
9 Gew.-% Sucrose-di-Laurat,
8,5 Gew.-% Sucrose-di-Palmitat.
7 Gew.-% Sucrose-mono-Stearat und
2,5 Gew.-% Sucrose-di-Stearat.

Dieser Zuckerester wird mit Ethanol unter Rühren bei Raumtemperatur im Temperaturbereich von 20°C bis einschließlich 25°C vermischt, so dass sich ein homogenes Gemisch bildet. Dabei kann eine leichte Erwärmung auf eine Temperatur im Bereich von 48°C bis 52°C erfolgen. Es wird so lange weiter gerührt, bis eine homogene Mischung beziehungsweise Lösung erreicht ist. Dann wird das Xantho-Flav Pulver zugegeben und unter Rühren auf eine Temperatur im Bereich von 83°C bis 97°C weiter erhitzt. Es wird derart stark gerührt, dass sich Xanthohumol homogen mit der vorgelegten Lösung verbindet. Das Solubilisat ist nach Abkühlen auf Raumtemperatur dunkelbraun und transparent. In diesem Zustand wird es abgefüllt und dann gelagert.

**Ausführungsbeispiel 7**

5 %iges Xantho Flav-Solubilisat

Es werden

| | |
|---|---|
| 50 g | Xantho-Flav Pulver, |
| 313,5 g | Zuckerester aus Speisefettsäuren (E 473) "Ryoto L-1695", |
| 313,5 g | Zuckerester aus Speisefettsäuren (E 473) "Ryoto P-1670" und |
| 323 g | Ethanol 96 %ig Neutralalkohol Sorte1411U |

verwendet.

Die beiden pulvrigen Zuckerester werden zusammen gegeben. Ein Mischvorgang ist für diesen Schritt nicht erforderlich. Die Zuckerester werden dann mit Ethanol unter Rühren bei Raumtemperatur im Temperaturbereich von 20°C bis einschließlich 25°C vermischt, so dass sich ein homogenes Gemisch bildet. Dieses wird unter Rühren auf eine Temperatur von maximal 80°C unter Rühren erwärmt. Es wird so lange weiter gerührt, bis eine homogene, transparente Lösung erreicht ist. Dann wird das Xantho-Flav Pulver zugegeben und unter Rühren auf eine Temperatur im Bereich von 83°C bis 97°C weiter erhitzt. Es wird derart stark gerührt, dass sich Xanthohumol homogen mit der vorgelegten Lösung verbindet. Das Solubilisat ist nach Abkühlen auf Raumtemperatur dunkelbraun und transparent. In diesem Zustand wird es abgefüllt und dann gelagert.

**Ausführungsbeispiel 8**

5 %iges Xantho Flav-Solubilisat

Es werden

| | |
|---|---|
| 50 g | Xantho-Flav Pulver, |
| 613,75 g | Zuckerester aus Speisefettsäuren (E 473) "Ryoto L-1695", |
| 318,25 g | Ethanol 96 %ig Neutralalkohol Sorte 1411U |

verwendet.

Die Herstellung des Solubilisats entspricht dem in Ausführungsbeispiel 7 beschriebenen Vorgehen mit einem Zuckerester alleine statt der dort verwendeten zwei.

**Ausführungsbeispiel 9**

5 % %iges Xantho Flav-Solubilisat

Es werden

| | |
|---|---|
| 50 g | Xantho-Flav Pulver, |
| 142,5 g | Zuckerester aus Speisefettsäuren (E 473) "Ryoto L-1695", |
| 332,5 g | Zuckerester aus Speisefettsäuren (E 473) "DUB SE 16 P", |
| 304 g | Ethanol 96 %ig Neutralalkohol Sorte 1411U und |
| 171 g | Glycerin |

verwendet.

Die beiden pulvrigen Zuckerester werden zusammengegeben. Ein Mischvorgang ist für diesen Schritt nicht erforderlich.

Die Zuckerester werden dann mit Ethanol und Glycerin unter so starkem Rühren bei Raumtemperatur im Temperaturbereich von 20°C bis einschließlich 25°C vermischt, dass sich ein homogenes Gemisch bildet. Dieses wird unter Rühren auf eine Temperatur von maximal 80°C unter Rühren erwärmt. Es wird so lange weiter gerührt, bis eine homogene, transparente Lösung erreicht ist. Dann wird das Xantho-Flav Pulver zugegeben und unter Rühren auf eine Temperatur im Bereich von 83°C bis 97°C weiter erhitzt. Es wird derart stark gerührt, dass sich Xanthohumol homogen mit der vorgelegten Lösung verbindet. Das Solubilisat ist nach Abkühlen auf Raumtemperatur dunkelbraun und transparent. In diesem Zustand wird es abgefüllt und dann gelagert.

**Ausführungsbeispiel 10**

5 % %iges Xantho Flav-Solubilisat

Es werden

| | |
|---|---|
| 50 g | Xantho-Flav Pulver, |
| 71,25 g | Zuckerester aus Speisefettsäuren (E 473) "Ryoto L-1695", |
| 166,25 g | Zuckerester aus Speisefettsäuren (E 473) "DUB SE 16 P", |
| 475 g | Polysorbat 80, |
| 152 g | Ethanol 96 %ig Neutralalkohol Sorte 1411U und |
| 82,5 g | Glycerin |

verwendet.

Die Zuckerester werden mit Polysorbat 80, Ethanol und Glycerin unter Rühren bei Raumtemperatur im Temperaturbereich von 20°C bis einschließlich 25°C vermischt, so dass sich ein homogenes Gemisch bildet. Dabei kann eine leichte Erwärmung auf eine Temperatur im Bereich von 48°C bis 52°C erfolgen. Es wird so lange weiter gerührt, bis eine homogene Mischung beziehungsweise Lösung erreicht ist. Dann wird das Xantho-Flav Pulver zugegeben und unter Rühren auf eine Temperatur im Bereich von 83°C bis 97°C weiter erhitzt. Es wird derart stark gerührt, dass sich Xanthohumol homogen mit der vorgelegten Lösung verbindet. Das Solubilisat ist nach Abkühlen auf Raumtemperatur dunkelbraun und transparent.

**Ausführungsbeispiel 11**

5 %iges Xantho Flav-Solubilisat

Es werden

| | |
|---|---|
| 50 g | Xantho-Flav Pulver, |
| 313,5 g | Zuckerester aus Speisefettsäuren (E 473) "Ryoto M-1695", |
| 313,5 g | Zuckerester aus Speisefettsäuren (E 473) "Ryoto P-1670", |
| 9,5 g | Lecithin als Phospholipid und |
| 313,5 g | Ethanol 96 %ig Neutralalkohol Sorte 1411U |

verwendet.

Die beiden pulvrigen Zuckerester werden zusammengegeben. Ein Mischvorgang ist für diesen Schritt nicht erforderlich. Das Lecithin wird mit Ethanol gemischt. Dabei kann eine leichte Erwärmung auf eine Temperatur im Bereich von 40°C bis 50°C erfolgen. Die Zuckerestermischung wird dann mit der Mischung aus Ethanol und Lecithin unter so starkem Rühren bei Raumtemperatur im Temperaturbereich von 20°C bis einschließlich 25°C vermischt, dass sich ein homogenes Gemisch bildet. Bei Bedarf kann dabei eine leichte Erwärmung auf eine Temperatur im Bereich von 40°C bis 50°C erfolgen. Diese kann auch durch die Zugabe des zuvor erwärmten Ethanol-Lecithin-Gemisches erfolgen. Das Gemisch wird unter Rühren auf eine Temperatur von maximal 80°C unter Rühren erwärmt. Es wird so lange weiter gerührt, bis eine homogene, transparente Lösung erreicht ist. Dann wird das Xantho-Flav Pulver zugegeben und unter Rühren auf eine Temperatur im Bereich von 83°C bis 97°C weiter erhitzt. Es wird derart stark gerührt, dass sich das Xantho-Flav-Pulver beziehungsweise das Xanthohumol homogen mit der vorgelegten Lösung verbindet. Das Solubilisat ist nach Abkühlen auf Raumtemperatur dunkelbraun und transparent. In diesem Zustand wird es abgefüllt und dann gelagert.

**Ausführungsbeispiel 12**

5 %iges Xantho Flav-Solubilisat

Es werden

| | |
|---|---|
| 50 g | Xantho-Flav Pulver, |
| 332,5 g | Zuckerester aus Speisefettsäuren (E 473) "Ryoto L-1695", |
| 142,5 g | Zuckerester aus Speisefettsäuren (E 473) "DUB SE 16 P", |
| 152 g | Ethanol 96 %ig Neutralalkohol Sorte 1411U, |
| 85,5 g | Glycerin und |
| 237,5 g | Wasser |

verwendet.

Die beiden pulvrigen Zuckerester werden zusammengegeben. Ein Mischvorgang ist für diesen Schritt nicht erforderlich. Die Zuckerester werden dann mit Ethanol, Wasser und Glycerin unter so starkem Rühren bei Raumtemperatur im Temperaturbereich von 20°C bis einschließlich 25°C vermischt, dass sich ein homogenes Gemisch bildet. Dieses wird unter Rühren auf eine Temperatur von maximal 80°C unter Rühren erwärmt. Es wird so lange weiter gerührt, bis eine homogene, transparente Lösung erreicht ist. Dann wird das Xantho-Flav Pulver zugegeben und unter Rühren auf eine Temperatur im Bereich von 83°C bis 97°C weiter erhitzt. Es wird derart stark gerührt, dass sich Xanthohumol homogen mit der vorgelegten Lösung verbindet. Das Solubilisat ist nach Abkühlen auf Raumtemperatur dunkelbraun und transparent. In diesem Zustand wird es abgefüllt und dann gelagert.

**Ausführungsbeispiel 13**

10 %iges Xantho Flav-Solubilisat

Es werden

| | |
|---|---|
| 100 g | Xantho-Flav Pulver, |
| 315 g | Zuckerester aus Speisefettsäuren (E 473) "Ryoto L-1695", |
| 135 g | Zuckerester aus Speisefettsäuren (E 473) "DUB SE 16 P", |
| 144 g | Ethanol 96 %ig Neutralalkohol Sorte 1411U, |
| 81 g | Glycerin und |
| 225 g | Wasser |

verwendet.

Die Herstellung des Solubilisats entspricht dem in Ausführungsbeispiel 12 beschriebenen Vorgehen.

Ob eine ausreichend vollständige Homogenisierung der Komponenten zu einem erfindungsgemäßen Solubilisat abgeschlossen ist, wird bei der Herstellung über Messungen der Klarheit des Produktes mit Hilfe eines Laserstrahls überprüft. Eine solche Laserstrahlmessung kann beispielsweise durch Beleuchten der Probe mit Hilfe eines handelsüblichen Laserpointers, insbesondere mit einer Wellenlänge im Bereich zwischen 650 nm und 1700 nm (Spektralfarbe Rot), und anschließender Sichtkontrolle des beleuchteten beziehungsweise durchleuchteten Solubilisats. Die Kontrolle wird nicht durch Probenahme und damit außerhalb des Reaktionskessels, sondern im Reaktionskessel durchgeführt. Der Laserstrahl wird durch ein Sichtglas, welches sich auf der Vorderseite des Reaktionskessels befindet, senkrecht zum Reaktionskessel gerichtet. Wenn auf der hinteren Innenseite des Reaktionskesses vollkommen frei von Streuung nur ein Lichtpunkt erscheint, sind die entstandenen Partikelstrukturen im Reaktionskessel kleiner als die Wellenlänge des sichtbares Lichtes und somit eine optische Bestätigung, dass der Prozess der Micellierung abgeschlossen ist.

Durch die gemessenen kleinen Partikelgrößen wird vorteilhafterweise die Ausbildung einer insbesondere für die Wahrnehmung mit dem menschlichen Auge klaren Flüssigkeit erreicht.

Die Klarheit des Solubilisats läßt sich auch durch seine geringe Trübung darstellen. Dazu wird folgende Arbeitshypothese angewandt: Je klarer eine wässrige Verdünnung eines Solubilisats oder einer anderen Formulierung von Xanthohumol, insbesondere unter physiologischen Bedingungen einer Magenpassage also bei einem pH-Wert von 1,1 und einer Temperatur von 37°C, ist, desto besser ist dessen Solubilisation. Je besser die Solubilisation, desto besser ist die Bioverfügbarkeit des Wirkstoffes beziehungsweise des ihn enthaltenden Produktes.

Diese lässt sich bereits an der besonders geringen Trübung des Solubilisats ablesen, welche sich als eine Art Kenngröße für die Bioverfügbarkeit verstehen lässt. Die Trübung der erfindungsgemäßen Solubilisate wurde bestimmt durch Streulichtmessung mit Infrarotlicht nach den Vorschriften der Norm ISO 7027.

Die erfindungsgemäße transparente und vollständig stabil wasserlösliche Formulierung weist, ohne Hilfsstoffe wie in Weich- und Hartgelatinekapseln, in gelatinefreien Kapseln (hart und/oder weich) und in flüssigen auf Wasser basierenden Endprodukten pH-unabhängig eine stabile Transparenz auf. Produkte mit derartiger Transparenz und Wasserlöslichkeit, werden seitens der relevanten Industrie dringend für innovative Produkte als Kapselfüllung gesucht. Eine Xanthohumolformulierung, die diesen Anforderungen gerecht wird, existiert bisher nach Kenntnis des Erfinders noch nicht.

Infolge der erfindungsgemäßen Formulierung in einem Solubilisat mit sehr kleinen, stabilen und magensaftresistenten Micellen schafft die Erfindung ein Solubilisat von Xanthohumol zur Verwendung als Nahrungsergänzungsmittel und/oder Arzneimittel, insbesondere zur Verwendung als Nahrungsergänzungsmittel und/oder Arzneimittel zur Behandlung von Adipositas.

Im Rahmen der Erfindung kann der Gehalt an Xanthohumol in den einzelnen Solubilisaten je nach Anwendungsfall auch deutlich höher als im gezeigten Beispiel eingestellt werden.

Werden höhere Beladungen mit Wirkstoff eingestellt, ist dies dadurch begrenzt, dass bei Überschreiten eines für die jeweilige Zusammensetzung individuellen Wirkstoffgehalts kein Solubilisat, sondern eine Emulsion hergestellt wird. Wird der Wirkstoffgehalt erhöht, sinken notwendigerweise die entsprechenden Anteile der anderen Komponenten (in Gew.-%). Oberhalb einer spezifischen Grenze erhält man ein disperses System, das jedoch nicht wie die erfindungsgemäßen Solubilisate irreversible in Wasser löslich ist und die für diese Solubilisate unter physiologischen Bedingungen der Magenpassage, also bei pH 1,1 und 37°C, gemessene sehr niedrige Trübung aufweist. Derartige Dispersionen können (Nano-)Emulsionen sein, sie sind aber keine Solubilisate, in denen der Wirkstoff oder die Wirkstoffe in den sehr kleinen Micellen eingeschlossen vorliegen. Nur die Solubilisate ermöglichen aber nach den Erfahrungen des Erfinders die deutlich erhöhte Bioverfügbarkeit des Wirkstoffs oder der Wirkstoffe gemäß der Erfindung, selbst wenn eine Emulsion eine höhere Wirkstoffbeladung erlaubte.

An der medizinischen Fakultät der Friedrich-Alexander Universität Erlangen Nürnberg (FAU) wurden Untersuchungen zur Wirkung von Xanthohumol auf das Körpergewicht durchgeführt. Es wurde eine Dosis von 2,5 mg/Kg Körpergewicht pro Tag Xanthohumol verabreicht. Dazu wurde ein Solubilisat der Anmelderin verwendet. Dieses wurde hergestellt aus 100 g "Xantho-Flav Pur", 150 g 96 %igem Ethanol und 750 g Polysorbat 80. Zum Einstellen der Konzentration auf 0,375 mg/mL wurde das Solubilisat mit doppelt-destiliertem Wasser ("ddH2O") verdünnt. In den beigefügten Figuren sind die entsprechenden Daten mit "s-XN" gekennzeichnet.

Zum Vergleich wurden entsprechende Experimente mit derselben Dosis an nativem Xanthohumol durchgeführt. Zum Lösen des nativen Xanthohumols wurde eine wässrige Lösung von MethylHydroxypropylcellulose (MHPC, 0,2 %, Methocel E4M Prmium CR, Hypromellose 2910 USP, Fragron Inc., Minnesota, USA) eingesetzt. Die entsprechenden Daten sind mit "n-XN" gekennzeichnet.

Die Lösungen wurden jeweils wöchentlich frisch hergestellt und lichtgeschützt und kühl gelagert.

Männliche Mäuse des Stammes C57BL/6 im Alter von 8 Wochen erhielten entweder übliche Nahrung oder wurden nach der "Western type diet" aus 15 % Schweineschmalz, 15 % Rindertalg, 4 % Palmitinsäure, 4 % Stearinsäure, 0,2 % Cholesterin und 30 % Saccharose ernährt. Bei der Kontrollgruppe (CTRL), welche übliche Nahrung erhielt, wurde als Therapie eine wässrige Lösung von Methylhydroxypropylcellulose (MHPC, 0,2 %, methocel™ E4M prem) gegeben ("Vehikel" VH), ebenso bei der "Western type diet"- Gruppe ("WTD").

Weitere Gruppen erhielten das Xanthohumol-Solubilisat ("s-XN") der Anmelderin oder natives Xanthohumol ("n-XN") zusätzlich zur WTD. Die Therapie wurde über 8 Wochen durchgeführt.

Nach sieben Wochen der Anwendung des Solubilisates oder des nativen Xanthohumols wurde ein intraperitonealer Glukosetoleranztest (ipGTT) durchgeführt. Nach 12 Stunden Fasten wurde den Mäusen intraperitoneal eine Glukoselösung in einer Glukosekonzentration von 50 % (Masse an Glukose pro Volumen) in einer Dosis von 3 mg Glukose pro g Körpergewicht injiziert. Die Konzentration an Glukose im Blut wurde gemessen in Proben, welche aus der Schwanzvene vor ("Nüchternglukosewert"; "fasting glucose") und nach 30, 60, 80 und 120 Minuten nach der Glukosegabe entnommen wurden. Die Messung wurde mit einem Glukometer des Modelles "accutrend" (Roche, Mannheim, Deutschland) durchgeführt.

Zur Quantifizierung von Leberlipiden wurden diese extrahiert und die Menge von Triglyceriden ("hepatic triglyceride level") mittels eines "GPO-triglyceride kit" (Sigma, Deisenhofen, Deutschland) bestimmt wie in "Wobser et al.: "Lipid accumulation in hepatocytes induces fibrogenic activation of hepatic stellate cells", Cell Res 2009; 19: 996 - 1005" beschrieben.

RNA-Isolation aus Lebergewebe, Reverstransskription ("reverse transccription") und quantitative Echtzeit-Polymerasekettenreaktionsanalyse ("quantitative real-time PCR analysis") wurden durchgeführt unter Anwendung der "LightCycler"-Technologie (Roche) und spezifischen Tests auf Primer wie in "Hellebrand et al.: "Promoter- hypermethylation is causing functional relevant downregulation of methylthioadenosine phosphorylase (MTAP) expression in hepatocellular carcinoma", Carcinogenesis 2006; 27: 64 - 72" beschrieben.

Für die histologische Analyse wurden Lebergewebeproben der Mäuse 24 Stunden lang in 4%igem Formalin bei Raumtemperatur fixiert, in aufsteigender Ethanolreihe getrocknet und in Paraffin eingebettet. Abschnitte dieser formalinfixierten Gewebeblöcken mit einer Dicke von 5 Mikrometern wurden mit Xylol deparaffinisiert und mit Hämatoxylin und Eosin gefärbt wie in "Dorn et al.: "Increased expression of c-Jun in nonalcoholic fatty liver disease", Lab Invest. 2014; 94: 394 - 408" beschrieben.

In den beigefügten Figuren sind die Ergebnisse der oben erläuterten Experimente graphisch dargestellt. Es zeigen:

Figuren 1A bis D      Effekte auf die Gewichtszunahme und die (viszerale) Fettansammlung *bei Gabe von Xanthohumol-Solubilisat (s-XN) oder nativem Xanthohumol (n-XN)* während WTD-Ernährung;

Figur IE und F      Effekte auf den Nüchternglukosewert und die Glukosetoleranz bei Gabe von Xanthohumol- Solubilisat (s-XN) oder nativem Xanthohumol (n-XN) während WTD-Ernährung;

Figuren 2      Effekte auf die Steatose, das Lebergewicht und die Triglyceridmenge in der Leber bei Gabe von Xanthohumol-Solubilisat (s-XN) oder nativem Xanthohumol (n-XN) während WTD-Ernährung;

Figuren 3      Effekte auf die Ausschüttung fibroseprovozierender Gene in der Leber ("hepatic expression of profibrogenetic genes" bei Gabe von Xanthohumol-Solubilisat (s-XN) oder nativem Xanthohumol (n-XN) während WTD-Ernährung; und

Figuren 4      Effekte auf die Ausschüttung Leberzirrhose triggernder Proteine bei Gabe von Xanthohumol- Solubilisat (s-XN) oder nativem Xanthohumol (n-XN) während WTD-Ernährung;

Die Angabe "*" in den Figuren bedeutet p < 0,05 im Vergleich zu den Daten für die "Therapie" mit dem Vehikel (VH). Die Angabe "#" in den Figuren bedeutet p < 0,05 im Vergleich zu den Daten für Kontrollgruppe (CTR).

An der Universität Kairo in der Fakultät für Pharmazie am Institut für Pharmakologie wurden von Herrn Prof. Dr. M. T. Khayyal weitere Untersuchungen zum anti-inflammatorischen Effekt von Xanthohumol, auch in Verbindung mit Curcumin, jeweils in nativer oder in gemäß der Erfindung solubilisierter Form durchgeführt. Die Ergebnisse dieser Versuche sind in den folgenden Figuren graphisch dargestellt. Es zeigen:

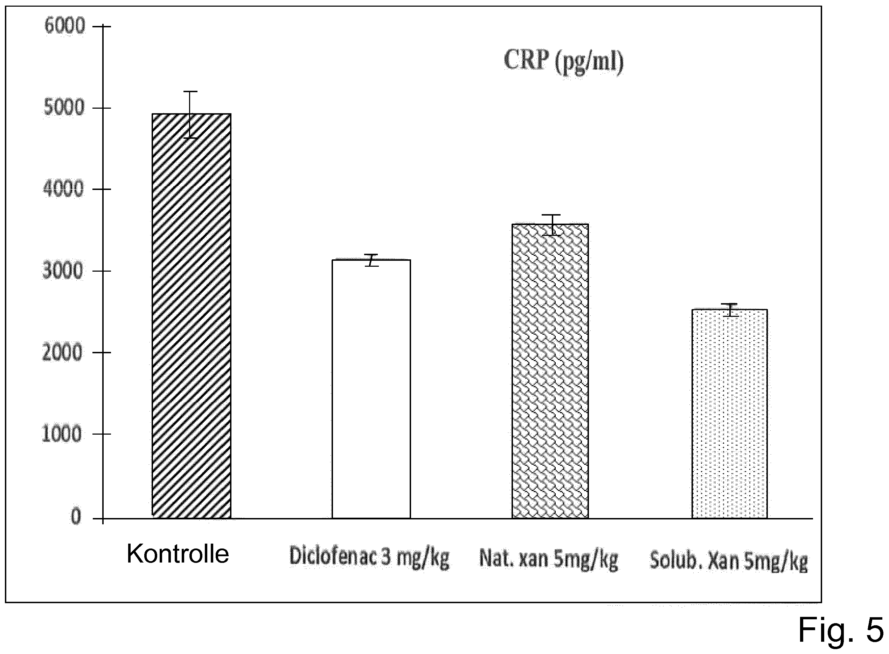

Figur 5      Effekt von Xanthohumol in nativer und solubilisierter Form auf den Serumgehalt an CRP (pg/L), und

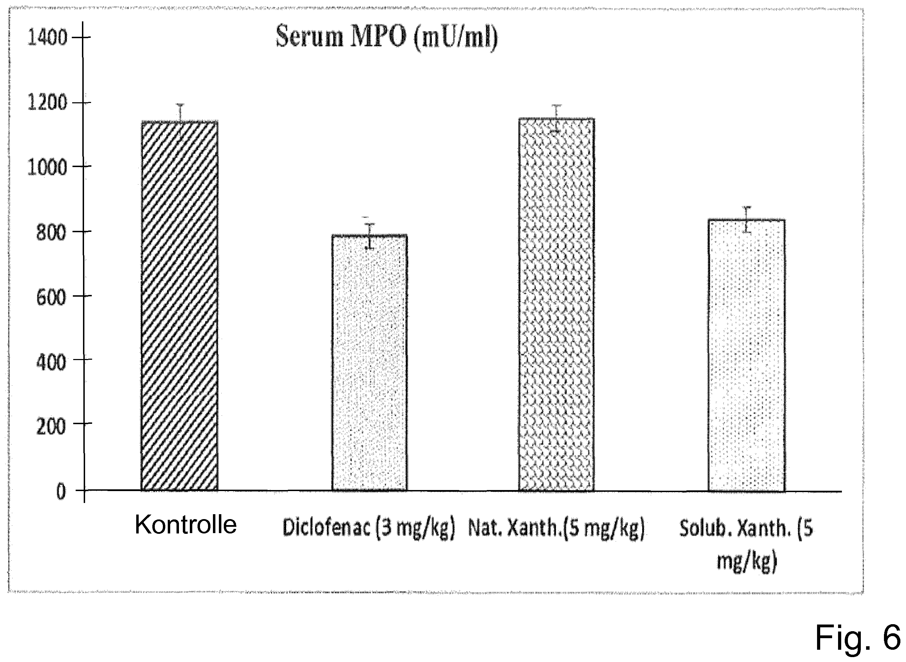

Figur 6      Effekt von Xanthohumol in nativer und solubilisierter Form auf den Serumgehalt an MPO (mU/mL).

Die WTD verursachte nicht nur Gewichtszunahme (Figur 1A), sondern auch eine signifikante Zunahme der Masse an visceralem und subkutanem Fettgewebe. Anhand fotografischer Aufnahmen der Versuchstiere gemäß Figuren 1C und ID zeigte sich, dass die Gabe des Xanthohumol-Solubilisats zu einer Verringerung der Masse an visceralem Fett führte.

Die Fütterung mit WTD führte des Weiteren zu einem höheren Nüchternglukosewert ("fasting glucose") und einer verringerten Glukosetoleranz verglichen mit Mäusen, welche mit der Kontrolle gefüttert wurden, was auf eine Insulinresistenz hinweist (vergleiche Figuren IE und 1F). Die Gabe des erfindungsgemäßen Xanthohumol-Solubilisats führte zu einer signifikanten Verbesserung der erhöhten Nüchternglukosewerte und der Glukosetoleranz, während natives Xanthohumol diesen Effekt nicht herbeiführte.

Im Ergebnis verbesserte die Anwendung von solubilisiertem Xanthohumol gemäß der Erfindung die durch die WTD induzierte Fettleibigkeit und Insulinresistenz.

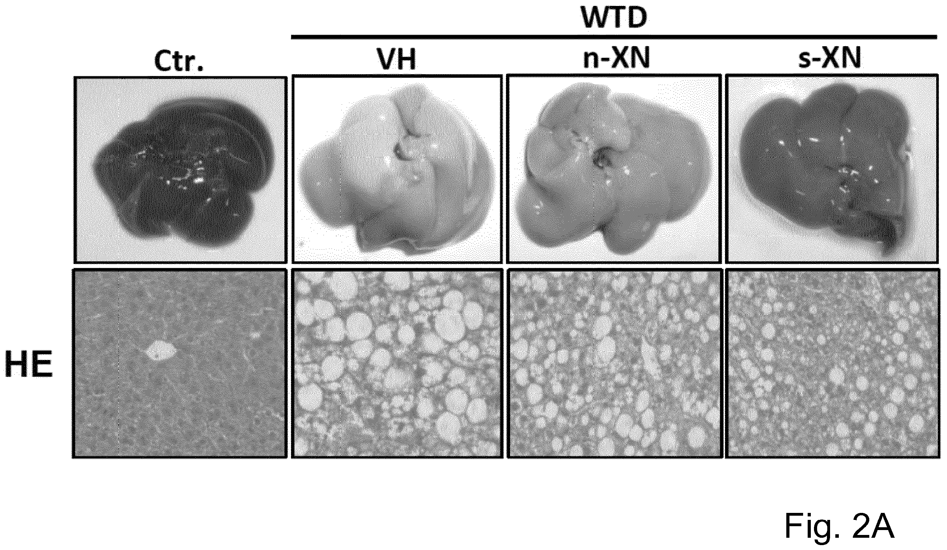

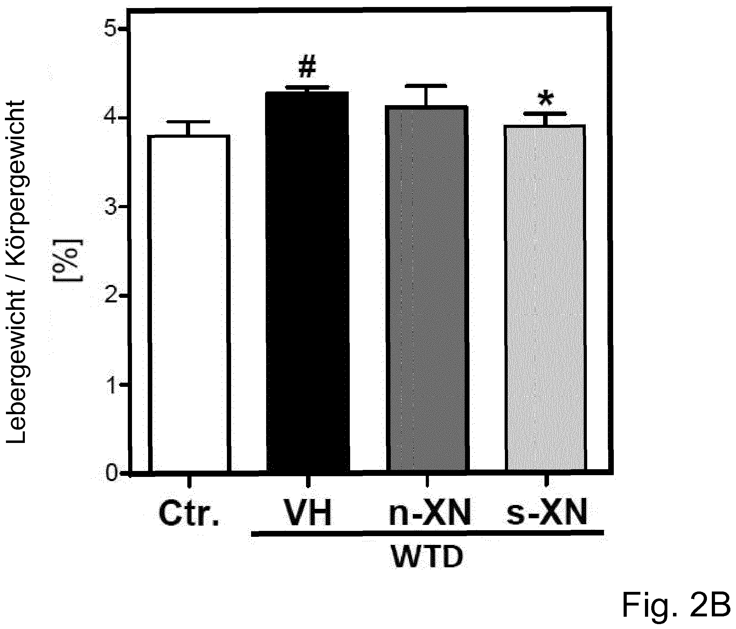

Des Weiteren verursachte die WTD eine Zunahme der Größe der Leber der Versuchstiere, welche sich sowohl anhand fotografischer Aufnahmen der Organe, welche am Ende der Versuchsreihe den Tieren entnommen wurden (Figur 2A), als auch an einer Zunahme des Gewichts der Leber zeigt (Figur 2B). Eine helle Färbung der Leber deutet zudem auf eine Fettansammlung in der Leber (Steatose) hin (Figur 2A für die Gabe des Vehikels und natives Xanthohumol). Die Gabe des erfindungsgemäßen Solubilisats zusätzlich zur WTD führte dazu, dass die Zunahme des Lebergewichts nahezu vollständig aufgehoben wurde, das heißt wieder den Ausgangswert erreichte (Figur 2B).

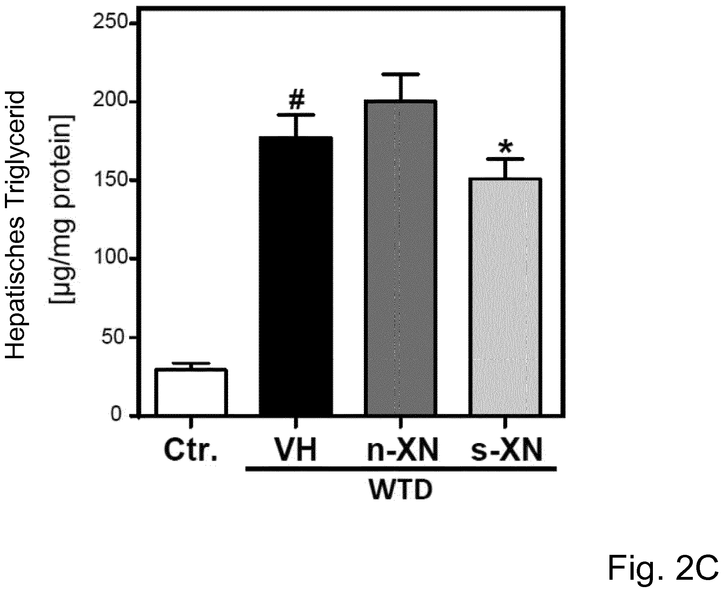

Die WTD führte zudem zu einem Anstieg des Triglyceridgehalts in der Leber. Auch die Histologie der Leber bestätigte den Befund Fettleber (hepatische Steatose). Die Gabe des erfindungsgemäßen Xanthohumol-Solubilisats gemeinsam mit der WTD führte zu einer bemerkenswerten Abnahme der Menge an Triglyceriden in der Leber (Figur 2C), welche sich ebenfalls bei histologischen Untersuchungen zeigte, bei welchen nur eine minimale Steatose im Vergleich zur Kontrollgruppe beobachtet wurde.

Die Marker einer Leberentzündung (Expression des proentzündlichen Chemokins MCP-1 und des Zytokins CXCL1) wurde durch die WTD signifikant erhöht im Vergleich zur Kontrolle (siehe Figuren 3A und 3B). Die Gabe des erfindungsgemäßen Xanthohumol-Solubilisats gemeinsam mit der WTD führte zu einem signifikanten Rückgang des Anstiegs dieser beiden Parameter (siehe Figur 3A).

Eine quantitative qPCR-Analyse der Lebern der mit der WTD gefütterten Mäuse zeigte eine erhöhte Expression des "alphasmooth muscle actins" (a-SMA), eines Proteins der glatten Muskulatur, (siehe Figur 4A). Das Protein a-SMA ist ein etablierter Marker aktivierter hepatischer Sternzellen (HSC), welche eine Leberzirrhose triggern. Eine immunhistochemische Analyse des Proteins a-SMA bestätigte, dass die WTD eine deutliche Aktivierung von HSC verursacht (Figur 4B). Diese geht einher mit einer signifikant erhöhten Expression des Kollagens Typ 1 (COL1A1) in der Leber von mit WTD gefütterten Mäusen im Vergleich zur Kontrollgruppe (Figur 4C). Kollagen Typ 1 (COL1A1) ist quantitativ und qualitativ das wichtigste extrazelluläre Matrixprotein bei der Leberfibrose.

Es ist bemerkenswert, dass die Behandlung mit dem erfindungsgemäß solubilisierten Xanthohumol die durch die WTD induzierte Expression von a-SMA und des Kollagens Typ 1 signifikant behindert, während natives Xanthohumol kaum Effekte zeigt (Figuren 4A, 4B und 4C).

Das erfindungsgemäße Solubilisat ist demnach dazu geeignet, eine sich bereits entwickelnde Leberentzündung, Steatose und Fibrose zurückzuführen.

Diese Befunde sind insbesondere deshalb überraschend, da die Xanthohumol-Gabe mit dem erfindungsgemäßen Solubilisat an bereits durch die zuvor erfolgte Ernährung nach der WTD geschädigte Organismen erfolgte. Es ist in biologischen Systemen beziehungsweise im Zusammenhang mit der Untersuchung von Wirkstoffen auf Krankheiten deutlich einfacher, eine Wirkung nachzuweisen, wenn der Wirkstoff von Beginn an zusammen mit dem Krankheitsinduzierenden Agens beziehungsweise Mechanismus, hier der WTD, verabreicht wird. Im Rahmen der oben beschriebenen Untersuchungen erfolgte die Gabe des erfindungsgemäßen Solubilisats dagegen erst bei bereits manifestierter Adipositas beziehungsweise bei bereits fortgeschrittener Organ-Schädigung, das heißt in der Therapiephase des Experiments.

Die den in weiteren Tierversuchen an Ratten ermittelten Dosen entsprechenden Dosierungen für den Menschen wurden wie unten beschrieben errechnet. Die Umrechnung der Dosierung für Ratten in die Dosierung für Menschen, jeweils in mg pro kg Körpergewicht und Tag, erfolgt nach der Gleichung

$$Dosierung_{Mensch} = \frac{Dosierung_{Ratte}}{37} \circ 6$$

Im Folgenden wird ein Beispiel erläutert für die Gabe von 5 mg Xanthohumol:

≙ 5 mg Xanthohumol/kg Körpergewicht Ratte

≙ 0,81 mg Xanthohumol/kg Körpergewicht Mensch.

Auf Basis eines Körpergewichts von 70 kg ergibt sich eine Tagesdosis für Menschen von:
÷ Xanthohumol:
((5 ÷ 37) x 6) x 70 = 56,76 mg Xanthohumol/Mensch/Tag

Für die Tagesdosis eines Menschen ergibt sich daraus folgende Solubilisat-Menge. Die folgende Berechnung wurde durchgeführt für ein 10 %iges Xanthohumol-Solubilisat.

Xanthohumol: 56,76 mg x 10 = 567,60 mg Solubilisat

Daraus ergibt sich für Polysorbat:
567,60 mg Solubilisat x 0,75 = 425,70 mg Polysorbat in der Gesamtmenge an Solubilisat.

Von einem Körpergewicht von 70 kg und der WHO-Empfehlung von einer Tagesaufnahme von 25 mg Polysorbat (= 1.750 mg Polysorbat/Tag) ausgehend, liegen die oben beschriebenen Solubilisate alle innerhalb der empfohlenen täglichen Aufnahmemenge der WHO.

An der Universität Kairo in der Fakultät für Pharmazie am Institut für Pharmakologie wurden von Herrn Prof. Dr. M. T. Khayyal Untersuchungen zum anti-inflammatorischen Effekt von Curcumin und von Kombinationen aus Curcumin mit Xanthohumol jeweils in nativer oder in gemäß der Erfindung solubilisierter Form durchgeführt.

Es wurden anti-inflammatorische Marker und die antioxidative Kapazität bestimmt. Weibliche Wistar-Ratten mit einem Körpergewicht zwischen 150 und 200 g wurden gemäß "Pearson et al. (1956)" der "Adjuvant induced arthritis" ausgesetzt. Den Tieren wurde über eine subplantare Injektion 0,1 ml Freund's Adjuvans (FCA) am Tag 0 in die rechte Hinterpfote verabreicht. Die Tiere wurden nach dem Zufallsprinzip in 12 Gruppen mit jeweils 8 Tieren aufgeteilt. Im Rahmen der Untersuchungen zum erfindungsgemäßen Xanthohumol-Solubilisat sind folgende der Gruppen interessant:

Gruppe 1 bildete die Kontrollgruppe.

Gruppe 2 erhielt Diclofenac als Referenzwirkstoff in einer Dosierung von 3 mg/kg Körpergewicht.

Gruppe 7 erhielt natives Xanthohumol in einer Dosierung von 5 m/kg Körpergewicht und

Gruppe 8 solubilisiertes Xanthohumol in derselben Dosierung.

Gruppe 12 erhielt eine Mischung aus solubilisiertem Curcumin und solubilisiertem Xanthohumol in jeweils derselben Dosierung.

Alle Extrakte bzw. Solubilisate wurden einmal täglich von Tag 0 bis Tag 21 nach der Impfung mit dem Adjuvans oral verabreicht. Nach Tag 21 wurden die Tiere getötet und Serumproben präpariert und bei - 80 °C gelagert. Gemessen wurden Myeloperoxidase (MPO), C-reaktives Protein (CRP), die gesamte antioxidative Kapazität (TAC) und die thiobarbitursäure-reaktiven Substanzen (thiobarbituratic acid reactive substances) (TBARS).

Die Ergebnisse der Untersuchungen werden im Folgenden anhand der beigefügten Figuren 5 und 6 erläutert.

Zunächst wurden die Auswirkungen auf das C-reaktive Protein (CRP) untersucht. C-reaktives Protein ist ein spezifischer Marker für eine antiinflammatorische Aktivität. Xanthohumol alleine hat einen vergleichbaren Effekt wie Diclofenac und zeigte eine bessere antiinflammatorische Aktivität in solubilisierter Form verglichen mit der nativen Form (Figur 5).

Myeloperoxidase (MPO) im Plasma spielt eine zentrale Rolle als pro-inflammatorischer Mediator in rheumatoider Arthritis und ist ein Indikator für das Einwandern neutrophiler Granulozyten in das betroffene Gewebe. Seine Konzentration ist bei Patienten mit rheumatoider Arthritis erhöht und verursacht oxidativen Stress. Die native Form von Xanthohumol selbst hatte nur einen geringen Effekt auf die Serumkonzentration an MPO, während die solubilisierte Form von Xanthohumol nahezu so effizient wie Diclofenac ist (Figur 6).

Oxidativer Stress ist einer der Hauptfaktoren, die bei rheumatoider Arthritis (RA) zur Gelenkzerstörung beitragen. Ein Anstieg der Produktion von sogenannten "reactive oxigen species (ROS)" führt zu einer verminderten Zufuhr von endogenen Antioxidantien und resultiert schließlich in der Zerstörung von Zellen. Die neutrophilen Granulozyten, welche im rheumatoiden Gelenk freigesetzt werden, produzieren freie Sauerstoffradikale, die zu einer erhöhten Bildung von Lipidperoxiden führen, welche sich in einem Anstieg im Serum TBARS zeigen. Daher kann der Anstieg im Antioxidantien-Status, welcher durch eine Zunahme im TAC repräsentiert wird, als Anzeige des Schutzes gegen die Entwicklung degenerativer entzündlicher Prozesse genutzt werden. Es gibt eine inverse Beziehung zwischen dem Level des TAC und dem des TBARS, ein hohes Level der antioxidativen Kapazität TAC korrespondiert zu einer geringen Konzentration von TBARS.

Die folgende Tabelle enthält Daten zum Effekt von Curcumin als Vergleich und von Xanthohumol im Hinblick auf die Erfindung in nativer und solubilisierter Form entweder alleine gegeben oder in Kombination mit Diclofenac in einer Dosierung von 3 mg/kg Körpergewicht einmal täglich 15 über 21 Tage auf die antioxidative Kapazität TAC und die thiobarbitursäure-reaktiven Substanzen TBARS im Serum arthritischer Ratten (n=8). Angegeben sind Mittelwerte ± Standardfehler SEM.

| Gruppe | TAC (nmol/Mikroliter) | TBARS (nmol/L) |
|---|---|---|
| Arthritische Kontrollgruppe | 57,26 ± 3,36 | 13,10 ± 0,39 |
| Diclofenac (3 mg/kg) | 82,08 ± 2,96 | 7,93 ± 0,84 |
| Natives Xanthohumol (5 mg/kg) | 63,71 ± 1,02 | 11,42 ± 1,16 |
| Solubilisiertes Xanthohumol (5 mg/kg) | 81,74 11,71 | 7,47 ± 0,53 |
| Natives Curcumin (5 mg/kg) + Xanthohumol (5 mg/kg) | 66,71 ± 1,27 | 11,74 ± 0,48 |
| Solubilisiertes Curcumin (5 mg/kg) + Xanthohumol (5 mg/kg) | 82,22 ± 1,53 | 7,90 ± 0,59 |

Gemäß der Erfindung solubilisiertes Xanthohumol alleine war dabei fast genauso effektiv wie Diclofenac bei der Reduzierung von TBARS und der Erhöhung des TAC im Serum der arthritischen Ratten, wie die Daten in der Tabelle zeigen.

Durch die gemessenen kleinen Partikelgrößen wird vorteilhafterweise die Ausbildung einer insbesondere für die Wahrnehmung mit dem menschlichen Auge klaren Flüssigkeit erreicht.

Die Klarheit des Solubilisats läßt sich auch durch seine geringe Trübung darstellen. Dazu wird folgende Arbeitshypothese angewandt: Je klarer eine wässrige Verdünnung eines Solubilisats oder einer anderen Formulierung von Xanhohumol, insbesondere unter physiologischen Bedingungen einer Magenpassage also bei einem pH-Wert von 1,1 und einer Temperatur von 37°C, ist, desto besser ist dessen Solubilisation. Je besser die Solubilisation, desto besser ist die Bioverfügbarkeit der Wirkstoffe beziehungsweise des sie enthaltenden Produktes.

Diese Bioverfügbarkeit lässt sich bereits an der besonders geringen Trübung des Solubilisats ablesen, welche sich als eine Art Kenngröße für die Bioverfügbarkeit verstehen lässt.

Die erfindungsgemäße transparente und vollständig stabil wasserlösliche Formulierung weist, ohne Hilfsstoffe wie in Weich- und Hartgelatinekapseln, in gelatinefreien Kapseln (hart und/oder weich) und in flüssigen auf Wasser basierenden Endprodukten pH-unabhängig eine stabile Transparenz auf. Produkte mit derartiger Transparenz und Wasserlöslichkeit, werden seitens der relevanten Industrie dringend für innovative Produkte als Kapselfüllung gesucht. Eine Formulierung von Xanthohumol, die diesen Anforderungen gerecht wird, existiert bisher nach Kenntnis des Erfinders noch nicht.

Infolge der erfindungsgemäßen Formulierung in einem Solubilisat mit sehr kleinen, stabilen und magensaftresistenten Micellen schafft die Erfindung ein Solubilisat von Xanthohumol zur Verwendung als Nahrungsergänzungsmittel und/oder Arzneimittel, insbesondere zur Verwendung als Nahrungsergänzungsmittel und/oder Arzneimittel mit antiinflammatorischer Wirkung.

Es ist dem Fachmann ersichtlich, dass die Erfindung nicht auf die vorstehend beschriebenen Beispiele beschränkt ist, sondern vielmehr in vielfältiger Weise variiert werden kann. Insbesondere können die Merkmale der einzeln dargestellten Beispiele auch miteinander kombiniert oder gegeneinander ausgetauscht werden.

## Patentansprüche

1. Wasserfreies Solubilisat bestehend aus,

   Xanthohumol mit einem Anteil von kleiner oder gleich 35 Gew.-%, bevorzugt kleiner oder gleich 15 Gew.-%, besonders bevorzugt 2 Gew.-% bis 12 Gew.-% und
   zumindest einem Emulgator mit einem HLB-Wert im Bereich zwischen 13 und 18, welcher insbesondere ausgewählt ist aus der Gruppe, welche Polysorbat 80, Polysorbat 20, Zuckerester aus Speisefettsäuren (E 473) sowie Phospholipide, insbesondere Lecithin, und Mischungen von zumindest zwei der genannten Emulgatoren umfasst,
   und bis zu 35 Gew.-%, bevorzugt bis zu 20 Gew.-%, besonders bevorzugt bis zu 15 Gew.-% Ethanol
   und optional bis zu 25 Gew.-%, bevorzugt bis zu 10 Gew.-% Glycerin.

2. Solubilisat nach Anspruch 1,
   **dadurch gekennzeichnet, dass** das Solubilisat als Quelle für Xanthohumol einen ethanolischen Auszug der Hartharze aus Hopfen enthält, wobei Xanthohumol in diesem Auszug in einer Konzentration im Bereich zwischen 60 Gew.-% und 95 Gew.-%, bevorzugt auf eine Konzentration im Bereich von 65 Gew.-% bis 85 Gew.-% vorliegt.

3. Solubilisat nach Anspruch 1 oder 2,
   **dadurch gekennzeichnet, dass**

   der Emulgator Polysorbat 80 oder Polysorbat 20 oder eine Mischung aus Polysorbat 20 und Polysorbat 80 ist, oder dass der Emulgator ein Zuckerester einer Speisefettsäure oder eine Mischung von zumindest zwei Zuckerestern von Speisefettsäuren ist,
   oder dass der Emulgator eine Mischung von Polysorbat 80 oder Polysorbat 20 oder einer Mischung aus Polysorbat 80 und Polysorbat 20 mit zumindest einem Zuckerester einer Speisefettsäure ist,
   oder dass der Emulgator eine Mischung zumindest eines Phospholipids, insbesondere Lecithin, mit zumindest einem Zuckerester einer Speisefettsäure ist.

4. Solubilisat nach einem der vorangegangenen Ansprüche, **dadurch gekennzeichnet, dass**
   das Verhältnis von Emulgator, insbesondere von Polysorbat 80, zu Xanthohumol im Bereich zwischen 30:1 und 3:1, bevorzugt im Bereich zwischen 25:1 und 5:1, bevorzugt im Bereich zwischen 9,8:1 bis 6,6:1 liegt.

5. Solubilisat nach einem der vorangegangenen Ansprüche, **dadurch gekennzeichnet, dass**
   der Emulgatoranteil, insbesondere der Polysorbatanteil, bei mindestens 45 Gew.-%, bevorzugt im Bereich zwischen 60 Gew.-% und 95 Gew.-%, besonders bevorzugt im Bereich zwischen 73 Gew.-% und 90 Gew.-% liegt.

6. Solubilisat nach einem der vorangegangenen Ansprüche, **dadurch gekennzeichnet, dass**
   die Durchmesserverteilung der Micellen in einer Verdünnung des Solubilisats mit destilliertem Wasser im Verhältnis 1:500 bei pH 1,1 und 37°C von etwa $d_{10}=70$ nm bis etwa $d_{90}=160$ nm reicht.

7. Solubilisat nach einem der vorangegangenen Ansprüche, **dadurch gekennzeichnet, dass**
die Trübung des Solubilisats kleiner als 100 FNU, bevorzugt kleiner als 50 FNU, ist gemessen durch Streulichtmessung mit Infrarotlicht nach den Vorschriften der Norm ISO 7027 bei einer Verdünnung des Solubilisats im Verhältnis 1:50 in Wasser bei pH 1,1 und 37°C.

8. Kapsel gefüllt mit einem wasserfreien Solubilisat nach einem der vorangegangenen Ansprüche,
**dadurch gekennzeichnet, dass**
die Kapsel als Weichgelatinekapsel oder Hartgelatinekapsel oder als weiche, gelatinefreie Kapsel oder als harte, gelatinefreie Kapsel, insbesondere als Cellulosekapsel, ausgebildet ist.

9. Fluid enthaltend ein wasserfreies Solubilisat nach einem der Ansprüche 1 bis 7,
**dadurch gekennzeichnet, dass**
das Fluid aus der Gruppe ausgewählt ist, welche Lebensmittel, Kosmetika und pharmazeutische Produkte umfasst.

10. Verfahren zum Herstellen eines Solubilisats nach einem der Ansprüche 1 bis 7,
mit folgenden Schritten

a) Vorlegen zumindest eines Emulgators mit einem HLB-Wert im Bereich zwischen 13 und 18, welcher insbesondere ausgewählt ist aus der Gruppe, welche Polysorbat 80, Polysorbat 20, Zuckerester aus Speisefettsäuren (E 473) und Phospholipide, insbesondere Lecithin, und Mischungen von zumindest zwei der genannten Emulgatoren umfasst,
b) Zugabe von Ethanol,
c) Erwärmen auf eine Temperatur bis zu 85°C, bevorzugt bis zu 80°C, unter Mischen beispielsweise durch Rühren,
d) Zugabe eines ethanolischen Auszugs der Hartharze aus Hopfen unter Mischen beispielsweise durch Rühren,

wobei in Schritt d) eine Erwärmung auf eine Temperatur im Bereich von 81°C bis 90°C, besonders bevorzugt auf eine Temperatur im Bereich von 83°C und 87°C erfolgt.

11. Verfahren nach Anspruch 10

wobei vor Schritt b) ein Schritt
b1) Lösen des ethanolischen Auszugs der Hartharze aus Hopfen in Ethanol unter Erwärmung auf eine Temperatur im Bereich von 40°C bis 62°C, bevorzugt auf eine Temperatur im Bereich von 45°C bis 57°C, besonders bevorzugt auf eine Temperatur im Bereich von 48°C und 52°C,
durchgeführt wird.

12. Verfahren nach Anspruch 11,
wobei in Schritt c) eine Erwärmung auf eine geringere Temperatur im Bereich von 40°C bis 62°C, bevorzugt auf eine Temperatur im Bereich von 45°C bis 57°C, besonders bevorzugt auf eine Temperatur im Bereich von 48°C und 52°C, erfolgt.

13. Verfahren nach Anspruch 12,
wobei in Schritt b) eine Zugabe eines Phospholipids, insbesondere eine Zugabe von Lecithin, zusammen mit Ethanol erfolgt.

14. Verfahren nach einem der Ansprüche 11 bis 13,
wobei in Schritt b) eine Zugabe von Glycerin zusammen mit Ethanol erfolgt.

**Claims**

1. An anhydrous solubilizate, consisting of

xanthohumol with a content of less than or equal to 35 wt.%, preferably less than or equal to 15 wt.%, most preferably 2 wt.% to 12 wt.%; and
at least one emulsifier with an HLB value in the range between 13 and 18, in particular selected from the group consisting of polysorbate 80, polysorbate 20, sucrose ester of edible fatty acids (E 473), and phospholipids, in particular lecithin, and mixtures of at least two of said emulsifiers;
and up to 35 wt.%, preferably up to 20 wt.%, most preferably up to 15 wt.% of ethanol;
and optionally up to 25 wt.%, preferably up to 10 wt.% of glycerol.

2. The solubilizate of claim 1,
**characterized in that**
the solubilizate contains an ethanolic extract of the hard resins from hops as a source for xanthohumol, with a concentration of xanthohumol in this extract in a range between 60 wt.% and 95 wt.%, preferably in the range from 65 wt.% to 85 wt.%.

3. The solubilizate of claim 1 or 2,
**characterized in that**

the emulsifier is polysorbate 80 or polysorbate 20 or a mixture of polysorbate 20 and polysorbate 80; or
the emulsifier is a sucrose ester of an edible fatty acid or a mixture of at least two sucrose esters of edible fatty acids; or
the emulsifier is a mixture of polysorbate 80 or polysorbate 20 or a mixture of polysorbate 80 and polysorbate 20 with at least one sucrose ester of an edible fatty acid; or
the emulsifier is a mixture of at least one phospholipid, in particular lecithin, with at least one sucrose ester of an edible fatty acid.

4. The solubilizate as claimed in any of the preceding claims, η
**characterized in that**
the ratio of emulsifier, in particular of polysorbate 80, to xanthohumol is in a range between 30:1 and 3:1, preferably in a range between 25:1 and 5:1, preferably in a range between 9.8:1 to 6.6:1.

5. The solubilizate as claimed in any of the preceding claims,
**characterized in that**
the emulsifier content, in particular the polysorbate content, is at least 45 wt.%, preferably in a range between 60 wt.% and 95 wt.%, most preferably in a range between 73 wt.% and 90 wt.%.

6. The solubilizate as claimed in any of the preceding claims,
**characterized in that**
the diameter distribution of the micelles in a dilution of the solubilizate with distilled water in a ratio of 1:500 at pH 1.1 and 37 °C ranges from about $d_{10}$ = 70 nm to about $d_{90}$ = 160 nm.

7. The solubilizate as claimed in any of the preceding claims,
**characterized in that**
the solubilizate has a turbidity of less than 100 FNU, preferably less than 50 FNU, as measured by scattered light measurement using infrared light in compliance with the specifications of the ISO 7027 standard at a dilution of the solubilizate in a ratio of 1:50 in water at pH 1.1 and 37 °C.

8. A capsule filled with an anhydrous solubilizate as claimed in any of the preceding claims,
**characterized in that**
the capsule is in the form of a soft gelatin capsule or a hard gelatin capsule or a soft gelatin-free capsule or a hard gelatin-free capsule, in particular a cellulose capsule.

9. A fluid, containing an anhydrous solubilizate as claimed in any of claims 1 to 7,
**characterized in that**
the fluid is selected from the group consisting of foods, cosmetics, and pharmaceutical products.

10. A method for producing a solubilizate according to any of claims 1 to 7, comprising the steps of

(a) providing at least one emulsifier with an HLB value in a range between 13 and 18, in particular selected from the group consisting of polysorbate 80, polysorbate 20, sucrose ester of edible fatty acids (E 473), and phospholipids, in particular lecithin, and mixtures of at least two of said emulsifiers;
(b) adding ethanol;
(c) heating to a temperature of up to 85 °C, preferably up to 80 °C, while mixing, for example by stirring;

(d) adding an ethanolic extract of the hard resins from hops while mixing, for example by stirring;

wherein step (d) comprises heating to a temperature in a range from 81 °C to 90 °C, most preferably to a temperature in a range from 83 °C to 87 °C.

11. The method of claim 10, wherein step (b) is preceded by a step
(b1) of dissolving in ethanol the ethanolic extract of the hard resins from hops while
heating to a temperature in a range from 40 °C to 62 °C, preferably to a temperature in a range from 45 °C to 57 °C, most preferably to a temperature in a range from 48 °C to 52 °C.

12. The method of claim 11, wherein
step (c) comprises heating to a lower temperature in a range from 40 °C to 62 °C, preferably to a temperature in a range from 45 °C to 57 °C, most preferably to a temperature in a range from 48 °C to 52 °C.

13. The method of claim 12, wherein
step (b) comprises adding a phospholipid, in particular adding lecithin together with ethanol.

14. The method as claimed in any of claims 11 to 13, wherein
step (b) comprises adding glycerol together with ethanol.

## Revendications

1. Solubilisat anhydre composée

de xanthohumol avec une proportion inférieure ou égale à 35% en poids, préférablement inférieure ou égale à 15 % en poids, de manière particulièrement préférable de 2 % en poids à 12 % en poids et
d'au moins un émulsifiant ayant une valeur HLB comprise entre 13 et 18, qui est notamment choisi dans le groupe comprenant le polysorbate 80, le polysorbate 20, des esters de sucre d'acides gras alimentaires (E 473) ainsi que des phospholipides, en particulier la lécithine, et des mélanges d'au moins deux des émulsifiants mentionnés,
et jusqu'à 35 % en poids, préférablement jusqu'à 20 % en poids, de manière particulièrement préférable jusqu'à 15 % en poids d'éthanol
et facultativement jusqu'à 25% en poids, préférablement jusqu'à 10 % en poids de glycérol.

2. Solubilisat selon la revendication 1,
**caractérisé en ce que** le solubilisat contient, comme source de xanthohumol, un extrait éthanolique des résines dures de houblon, le xanthohumol étant présent dans cet extrait dans une concentration comprise entre 60 % en poids et 95 % en poids, préférablement dans une concentration comprise entre 65 % en poids et 85 % en poids.

3. Solubilisat selon la revendication 1 ou 2,
**caractérisé en ce que**

l'émulsifiant est le polysorbate 80 ou le polysorbate 20 ou un mélange de polysorbate 20 et de polysorbate 80, ou **en ce que** l'émulsifiant est un ester de sucre d'un acide gras alimentaire ou un mélange d'au moins deux esters de sucre d'acides gras alimentaires,
ou **en ce que** l'émulsifiant est un mélange de polysorbate 80 ou de polysorbate 20 ou un mélange de polysorbate 80 et de polysorbate 20 avec au moins un ester de sucre d'un acide gras alimentaire,
ou **en ce que** l'émulsifiant est un mélange d'au moins un phospholipide, en particulier la lécithine, avec au moins un ester de sucre d'un acide gras alimentaire.

4. Solubilisat selon l'une des revendications précédentes,
**caractérisé en ce que**
le rapport entre l'émulsifiant, en particulier le polysorbate 80, et le xanthohumol est compris entre 30:1 et 3:1, préférablement entre 25:1 et 5:1, préférablement entre 9,8:1 et 6,6:1.

5. Solubilisat selon l'une des revendications précédentes,
**caractérisé en ce que**

la proportion d'émulsifiant, en particulier la proportion de polysorbate, est d'au moins 45 % en poids, préférablement comprise entre 60 % en poids et 95 % en poids, de manière particulièrement préférable comprise entre 73 % en poids et 90 % en poids.

6. Solubilisat selon l'une des revendications précédentes,
   **caractérisé en ce que**
   la répartition des diamètres des micelles dans une dilution du solubilisat avec de l'eau distillée dans un rapport de 1:500 avec un pH de 1,1 et une température de 37°C va d'environ $d_{10}$=70 nm à environ $d_{90}$=160 nm.

7. Solubilisat selon l'une des revendications précédentes,
   **caractérisé en ce que**
   la turbidité du solubilisat est inférieure à 100 FNU, préférablement inférieure à 50 FNU, mesurée par diffusion de la lumière à l'aide d'une lumière infrarouge selon les prescriptions de la norme ISO 7027 pour une dilution du solubilisat dans un rapport de 1:50 dans de l'eau avec un pH de 1,1 et une température de 37°C.

8. Capsule remplie d'un solubilisat anhydre selon l'une des revendications précédentes,
   **caractérisée en ce que**
   la capsule est conçue comme une capsule de gélatine molle ou une capsule de gélatine dure ou comme une capsule molle, exempte de gélatine, ou comme une capsule dure, exempte de gélatine, en particulier une capsule de cellulose.

9. Fluide contenant un solubilisat anhydre selon l'une des revendications 1 à 7,
   **caractérisé en ce que**
   le fluide est choisi dans le groupe comprenant les produits alimentaires, les produits cosmétiques et les produits pharmaceutiques.

10. Procédé de préparation d'un solubilisat selon l'une des revendications 1 à 7,
    comprenant les étapes suivantes :

    a) présentation au moins d'un émulsifiant ayant une valeur HLB comprise entre 13 et 18, qui est notamment choisi dans le groupe comprenant le polysorbate 80, le polysorbate 20, des esters de sucre d'acides gras alimentaires (E 473) ainsi que des phospholipides, en particulier la lécithine, et des mélanges d'au moins deux des émulsifiants mentionnés,
    b) ajout d'éthanol,
    c) chauffage à une température jusqu'à 85°C, préférablement jusqu'à 80°C, par un mélange, par exemple en remuant,
    d) ajout d'un extrait éthanolique de résines dures de houblon, par un mélange, par exemple en remuant,

    à l'étape d), un chauffage à une température comprise entre 81°C et 90°C, de manière particulièrement préférable à une température comprise entre 83°C et 87°C, étant effectué.

11. Procédé selon la revendication 10,

    avant l'étage d), une étape
    b1) dissolution de l'extrait éthanolique des résines dures de houblon dans l'éthanol par un chauffage à une température comprise entre 40°C et 62°C, préférablement à une température comprise entre 45°C et 57°C, de manière particulièrement préférable à une température comprise entre 48°C et 52°C,
    étant effectuée.

12. Procédé selon la revendication 11,
    à l'étape c), un chauffage à une température plus faible, comprise entre 40°C et 62°C, préférablement à une température comprise entre 45°C et 57°C, de manière particulièrement préférable à une température comprise entre 48°C et 52°C, étant effectué.

13. Procédé selon la revendication 12,
    à l'étape b), un ajout d'un phospholipide, en particulier un ajout de lécithine, avec de l'éthanol, étant effectué.

14. Procédé selon l'une des revendications 11 à 13,
    à l'étape b), un ajout de glycérol, avec de l'éthanol, étant effectué.

Fig. 1B

Fig. 1A

22

Fig. 1C

Ctr. | WTD
VH | n-XN | s-XN

Fig. 1D

Fig. 1E

EP 3 820 527 B1

Fig. 1F

EP 3 820 527 B1

Fig. 2A

Fig. 2B

Fig. 2C

EP 3 820 527 B1

**Fig. 3B**

CXCL1 mRNA [Ctr. set1]

Ctr. | VH | n-XN | s-XN
WTD

**Fig. 3A**

MCP1 mRNA [Ctr. set1]

Ctr. | VH | n-XN | s-XN
WTD

Fig. 3C

Fig. 4A

WTD

| Ctr. | VH | n-XN | s-XN |

Fig. 4B

Fig. 4C

EP 3 820 527 B1

CRP (pg/ml)

| | |
|---|---|
| 6000 | |
| 5000 | |
| 4000 | |
| 3000 | |
| 2000 | |
| 1000 | |
| 0 | |

Kontrolle    Diclofenac 3 mg/kg    Nat. xan 5mg/kg    Solub. Xan 5mg/kg

Fig. 5

Serum MPO (mU/ml)

Fig. 6

EP 3 820 527 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1431385 A1

**In der Beschreibung aufgeführte Nicht-Patentliteratur**

- **WOBSER et al.** Lipid accumulation in hepatocytes induces fibrogenic activation of hepatic stellate cells. *Cell Res,* 2009, vol. 19, 996-1005
- **HELLEBRAND et al.** Promoter- hypermethylation is causing functional relevant downregulation of methylthioadenosine phosphorylase (MTAP) expression in hepatocellular carcinoma. *Carcinogenesis,* 2006, vol. 27, 64-72
- **DORN et al.** Increased expression of c-Jun in nonalcoholic fatty liver disease. *Lab Invest.,* 2014, vol. 94, 394-408
- **PEARSON et al.** *Adjuvant induced arthritis,* 1956